(19) Europäisches Patentamt
European Patent Office
Office européen des brevets

(11) Veröffentlichungsnummer: **0 044 927**
**B1**

(12) **EUROPÄISCHE PATENTSCHRIFT**

(45) Veröffentlichungstag der Patentschrift: **28.11.84**

(21) Anmeldenummer: **81104475.9**

(22) Anmeldetag: **11.06.81**

(51) Int. Cl.³: **C 07 D519/00**, C 07 D493/04, A 61 K 31/52// C07D473/08, C07D473/10, C07D473/06, (C07D493/04, 307/00, 307/00),(C07D519/00, 493/00, 473/00)

(54) **Durch Purinbasen substituierte Alkylamino-desoxy-1.4 ; 3.6-dianhydro-hexit-nitrate, Verfahren zu ihrer Herstellung und pharmazeutische Zubereitung.**

(30) Priorität: **25.07.80 DE 3028272**

(43) Veröffentlichungstag der Anmeldung: **03.02.82 Patentblatt 82/05**

(45) Bekanntmachung des Hinweises auf die Patenterteilung: **28.11.84 Patentblatt 84/48**

(84) Benannte Vertragsstaaten: **AT BE CH FR GB IT LI NL SE**

(56) Entgegenhaltungen:
**US-A- 3 886 186**
**US-A- 4 065 488**
**EUROPEAN JOURNAL OF MEDICINAL CHEMISTRY, CHIMICA THERAPEUTICA, Band 15, Heft 1, Januar 1980, PARIS (FR) G. GIOVANNINETTI et al.: "Synthèse et activité hypocholestérolémique de bis-homoanalogues des nucléosides puriques", Seiten 23-27**
**CHEMICAL ABSTRACTS, Band 92, 1980, Seiten 627, Zusammenfassung 147106x, COLUMBUS, OHIO (US) P. ANGIBEAUD et al.: "Homoanalogs of nucleosides"**
**CHEMICAL ABSTRACTS, Band 87, 1977, Seiten 513, Zusammenfassung 6280j, COLUMBUS, OHIO (US) Y. ISHIDO et al.: "Synthetic studies by the use of carbonates. Part IX: Reactions of D-mannitol carbonate derivatives catalyzed by tetraethylammonium bromide"**
**CHEMICAL ABSTRACTS, Band 79, 1973, Zusammenfassung 42792w, COLUMBUS, OHIO (US) G. GIOVANNINETTI et al.: "Homoanalogs of aldofuranosyl nucleosides"**

(73) Patentinhaber: **Dr. Willmar Schwabe GmbH & Co.**
**Willmar-Schwabe-Strasse 4**
**D-7500 Karlsruhe 41 (DE)**

(72) Erfinder: **Klessing, Klaus, Dr. Dipl.-Chem.**
**Rheingoldstrasse 3**
**D-7505 Ettlingen 5 (DE)**
Erfinder: **Chatterjee, Shyam Sunder, Dr. Dipl.-Chem.**
**Werrabronner Strasse 8a**
**D-7500 Karlsruhe 41 (DE)**

(74) Vertreter: **Bunke, Holger, Dr. Dipl.-Chem. et al**
**Patentanwälte Prinz, Bunke & Partner Lessingstrasse 9**
**D-7000 Stuttgart 1 (DE)**

**EP 0 044 927 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft durch Purinbasen substituierte Alkylamino-deoxy-1.4 ; 3.6-dianhydro-hexitnitrate der allgemeinen Formel I,

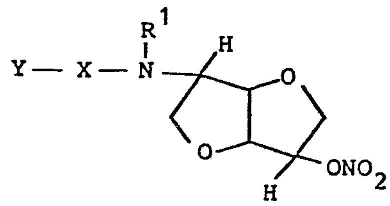

(I)

worin

$R^1$ ein Wasserstoffatom oder eine Niedrigalkylgruppe mit 1 bis 4 C-Atomen,

X eine geradkettige oder verzweigte Alkylen- oder Hydroxyalkylengruppe mit 1 bis 7 C-Atomen und

Y eine 1.3-Dialkylxanthin-7-yl- oder 3.7-Dialkylxanthin-1-yl-Gruppe, jeweils mit gerad- oder verzweigtkettigen Alkylgruppen mit 1 bis 5 C-Atomen, oder eine Adenin-9-yl-Gruppe

bedeuten, sowie deren physiologisch unbedenkliche Säureadditionssalze.

Das Grundgerüst dieser Verbindungen besteht aus einem der stereoisomeren, unter Epimerisierung ineinander umwandelbaren 1.4 ; 3.6-Dianhydro-hexite, nämlich entweder dem 1.4 ; 3.6-Dianhydro-L-idit (= « Isoidid ») (II),

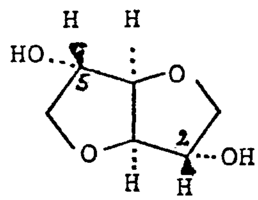

(II)

bei dem die OH-Gruppen in 2- und 5-Stellung jeweils exo-Konfiguration aufweisen, oder dem 1.4 ; 3.6-Dianhydro-D-glucit (=« Isosorbid ») (III),

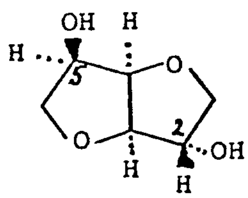

(III)

der eine 2-exo-ständige und eine 5-endo-ständige OH-Gruppe aufweist und somit — bei verschiedenen Substituenten in 2- und 5-Stellung — in zwei isomeren Formen auftritt.

Das Grundgerüst einiger Verbindungen schließlich besteht aus dem 1.4 ; 3.6-Dianhydro-D-mannit (=« Isomannid ») (IV),

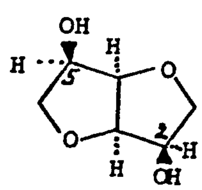

(IV)

der zwei endo-ständige OH-Gruppen aufweist.

Da im Gegensatz zu den Glucit-Derivaten bei den Idit- und Mannit-Derivaten eine Unterscheidung zwischen den 2- und 5-Substituenten nicht möglich ist, weil das $C^2$-Atom bei Drehung des Moleküls um 180° zum $C^5$-Atom wird, sind Hinweise auf die 5-Stellung oder 2-Stellung von Substituenten bei diesen Derivaten an sich überflüssig. Des besseren Vergleiches der Strukturen der einzelnen Verbindungen mit den allgemeinen Formeln wegen werden aber hier die Isoidid-Derivate durchweg als 5-Amino-isoidid-Derivate bezeichnet, da sie aus den in 5-Stellung acylsubstituierten Isosorbid-Derivaten entstehen. Entsprechend werden die als Ausgangsverbindungen verwendeten Isomannid-Acylderivate als 2-Acylisomannid-Derivate bezeichnet, da aus ihnen die in 2-Stellung substituierten Isosorbid-Derivate hergestellt werden.

Eine kurze Zusammenfassung über die Stereoisomerie der 1.4 ; 3.6-Dianhydro-hexite gibt J.A. Mills in Advances in Carbohydrate Chem. *10,* 1-53 (1955).

Die Erfindung betrifft weiter Verfahren zur Herstellung der eingangs genannten, durch Purinbasen substituierten Alkylamino-1.4 ; 3.6-dianhydro-hexit-mononitrate sowie pharmazeutische Zubereitungen, die die erfindungsgemäßen Verbindungen enthalten.

Die Nitrate des 1.4 ; 3.6-Dianhydro-D-glucits (auch 1.4 ; 3.6-Dianhydro-D-sorbit genannt) sind z. B. aus der US-A-3 886 186 bekannt, und zwar sowohl die 2- und 5-Mononitrate als auch das 2,5-Dinitrat des Isosorbids. Diese Nitrate, insbesondere das Dinitrat, das bereits als Arzneimittel im Handel ist, sind pharmakologisch wirksame Substanzen mit hämodynamischer, vasodilatorischer und antianginöser Wirksamkeit, die insbesondere bei Koronarinsuffizienz und zur Behandlung von Angina Pectoris verwendet werden.

Aus der US-A-4 065 488 ist ein Verfahren zur Herstellung von Isosorbid-mononitrat durch Acetylierung von Isosorbid mit Essigsäureanhydrid, nachfolgende Nitrierung und anschließende hydrolytische Abspaltung des 2- bzw. 5-Acetylrests bekannt. Durch Purinbasen substituierte 1.4 ; 3.6-Dianhydro-hexitnitrate und Verfahren zu deren Herstellung sind dagegen nicht beschrieben.

Aus Eur. J. Med. Chem. *15,* 23 (1980) sind durch Purinbasen substituierte 1.4- oder 3.6-Monoanhydrohexit-derivate, jedoch keine Nitrate, und deren Herstellung bekannt. Diese Verbindungen weisen den Cholesterinspiegel senkende Aktivität auf.

Vergleichbare, durch Purinbasen substituierte Monohexit-derivate mit freien Hydroxylgruppen sind auch aus Chemical Abstracts *79,* 42792w (1973) ; *87,* 6280j (1977) ; und *92,* 147106x (1980) bekannt.

Irgendwelche Hinweise auf die erfindungsgemäß beanspruchten Verbindungen und die Verfahren zu ihrer Herstellung lassen sich diesen Druckschriften nicht entnehmen.

Die Pharmakokinetik des Dinitrats und der Mononitrate von Isosorbid, Isomannid und Isoidid wurde von Bogaert und Rosseel in Naunyn-Schmiedeberg's Arch. Pharmacol. *275,* 339 (1972) beschrieben.

Es hat sich jedoch gezeigt, daß die Nitrate unangenehme Nebenwirkungen, insbesondere Kopfschmerzen, verursachen. Die Mononitrate sind außerdem schlechter resorbierbar als etwa das Isosorbiddinitrat (ISDN). Es kommt hinzu, daß sich die Dinitrate des Isosorbids, Isomannids und Isoidids nur unter besonderen Vorsichtsmaßnahmen herstellen und handhaben lassen, da sie explosiv sind.

Somit bestand ein Bedürfnis nach der Bereitstellung neuer pharmazeutischer Mittel mit gleichem Wirkungsspektrum, die jedoch die genannten Nachteile nicht aufweisen, und nach der Schaffung neuer 1.4 ; 3.6-Dianhydro-hexit-mononitrate, die als wirksame Bestandteile solcher pharmazeutischer Mittel verwendet werden können.

Die der Erfindung zugrundeliegende Aufgabe besteht darin, das angeführte Bedürfnis zu befriedigen, die Lösung dieser Aufgabe darin, die erfindungsgemäßen Stoffe bereitzustellen.

Gegenstand der Erfindung sind demnach

1. durch Purinbasen substituierte 5-Alkylamino-5-desoxy-1.4 ; 3.6-dianhydro-L-idit-2-nitrate der allgemeinen Formel V,

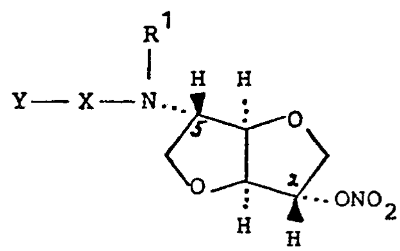

(V)

worin $R^1$ ein Wasserstoffatom oder eine Niedrigalkylgruppe mit 1 bis 4 C-Atomen,

X eine geradkettige oder verzweigte Alkyl- oder Hydroxyalkylgruppe mit 1 bis 7 C-Atomen,

Y eine 1.3-Dialkylxanthin-7-yl- oder 3.7-Dialkylxanthin-1-yl-Gruppe, jeweils mit gerad- oder verzweigtkettigen Alkylgruppen mit 1 bis 5 C-Atomen, oder eine Adenin-9-yl-Gruppe

bedeuten, sowie deren physiologisch unbedenkliche Säureadditionssalze ;

2. durch Purinbasen substituierte 5-Alkylamino-5-desoxy-1.4 ; 3.6-dianhydro-D-glucit-2-nitrate der allgemeinen Formel VI,

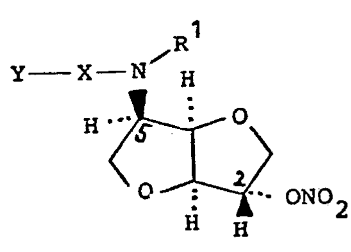

(VI)

worin $R^1$, X und Y die unter (1.) genannten Bedeutungen besitzen, sowie deren physiologisch unbedenkliche Säureadditionssalze ;

3. durch Purinbasen substituierte 2-Alkylamino-2-desoxy-1.4 ; 3.6-dianhydro-D-glucit-5-nitrate der allgemeinen Formel VII,

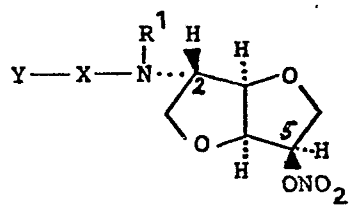

(VII)

worin $R^1$, X und Y die unter (1.) angegebenen Bedeutungen besitzen, sowie deren physiologisch unbedenkliche Säureadditionssalze ;

4. durch Purinbasen substituierte 5-Alkylamino-5-desoxy-1.4 ; 3.6-dianhydro-D-mannit-2-nitrate der allgemeinen Formel VIII,

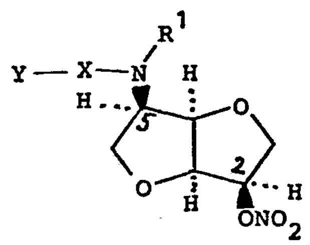

(VIII)

worin $R^1$, X und Y die unter (1.) genannten Bedeutungen besitzen, sowie deren physiologisch unbedenkliche Säureadditionssalze.

Die erfindungsgemäßen Verbindungen besitzen koronardurchflußsteigernde, spasmolytische, blutdrucksenkende, negativ inotrope und die Herzfrequenz senkende sowie z. Teil antiarrhythmische und die Thrombozytenaggregation hemmende Wirksamkeit. Sie sind zur Behandlung von Koronarerkrankungen, zur Kupierung und Prophylaxe von Angina-Pectoris-Anfällen, zur Nachbehandlung von Herzinfarkten und zur Behandlung besitzen eine gute therapeutische Breite. Die orale Absorption ist besonders gut und die Wirkungsdauer lang. Darüber hinaus bewirken sie eine Verbesserung der peripheren Durchblutung und der Gehirndurchblutung.

Die Handhabung und Herstellung der erfindungsgemäßen Verbindungen ist weit weniger gefährlich als etwa beim bekannten ISDN, weil sie nicht explosiv sind.

Die erfindungsgemäßen Verbindungen besitzen im 1.4 ; 3.6-Dianhydro-hexit-Grundgerüst 4 asymmetrische C-Atome und liegen in optisch aktiver Form vor, da als Ausgangsverbindungen optisch reine 1.4 ; 3.6-Dianhydro-hexite verwendet werden, die aus natürlich vorkommenden Zuckeralkoholen leicht zugänglich sind.

Die erfindungsgemäßen Verbindungen können ausgehend von den epimeren, unsubstituierten 1.4 ; 3.6-Dianhydro-hexiten, also ausgehend von L-Isoidid, D-Isosorbid und D-Isomannid, hergestellt werden, wobei im Falle des D-Isosorbids als Ausgangsverbindung zwei verschiedene Synthesewege möglich sind.

Der erste Weg besteht erfindungsgemäß darin, daß der entsprechende 1.4 ; 3.6-Dianhydro-hexit mit einem Sulfonsäurechlorid, vorzugsweise mit Methansulfonsäurechlorid oder Toluolsulfonsäurechlorid, in einem geeigneten wasserfreien Lösungsmittel und in Gegenwart einer Hilfsbase, vorzugsweise in Pyridin oder in Chloroform/Triäthylamin, bei erniedrigter Temperatur, vorzugsweise zwischen − 20° und + 10 °C, in den entsprechenden Mono-O-acyl-1.4 ; 3.6-dianhydrohexit übergeführt wird, welcher dann durch Zusatz einer wäßrigen, beispielsweise 25 %igen Ammoniaklösung oder durch Zusatz eines primären Alkylamins mit 1 bis 4 C-Atomen gegebenenfalls unter Zusatz eines geeigneten Lösungsmittels, einer Aminolyse unterworfen wird, und zwar vorteilhaft unter erhöhtem Druck, vorzugsweise bei einem Druck von 2-20 at, und erhöhter Temperatur, vorzugsweise bei 90 bis 180 °C. Die Aminolyse wird zweckmäßig in einem geschlossenen Stahlautoklaven bis zur quantitativen Umsetzung durchgeführt. Geeignete Lösungsmittel sind — gegebenenfalls unter Zusatz von Wasser — z. B. Alkohole, Di- oder Polyglykoläther oder Dioxan.

Bei der Aminolyse zum entsprechenden, gegebenenfalls N-monoalkylsubstituierten, Amino-desoxy-1.4 ; 3.6-dianhydrohexit wird die Mesylat- bzw. Tosylatgruppe durch die Amino- oder eine Monoalkylaminogruppe mit 1 bis 4 C-Atomen nach dem Reaktionsmuster einer typischen bimolekularen nucleophilen Substitution ($S_N2$-Reaktion) ausgetauscht, die stets mit einer Umkehr der Konfiguration am zentralen

Kohlenstoffatom verbunden ist. Diese Konfigurationsumkehr, dem Fachmann auch unter den Begriffen « Inversion » oder « Walden-Umkehr » geläufig, ist der Grund dafür, daß aus dem 1.4 ; 3.6-Dianhydro-D-glucit-5-acyl-Derivat, bei dem der Acylrest in 5-Stellung endo-ständig vorliegt, stets das in 5-Stellung durch die Aminogruppe oder eine Monoalkylaminogruppe mit 1 bis 4 C-Atomen substituierte 1.4 ; 3.6-Dianhydro-L-idit-Derivat entsteht, bei dem der anstelle des Acylrests in das Molekül eingetretene Substituent nicht mehr in endo-sondern in exo-Stellung steht. Die mit der $S_N2$-Reaktion verbundene Walden-Umkehr ist in ganz entsprechender Weise verantwortlich dafür, daß aus dem entsprechenden Idit-Acylat stets das in 5-Stellung endo-substituierte Glucit-Derivat, aus dem Mannit-Acylat das entsprechende, in 2-Stellung exo-substituierte Glucit-Derivat und aus dem Glucit-2-exo-acylat das entsprechende, in 2-Stellung endosubstituierte Mannit-Derivat entsteht.

Die im Verlauf des bisher beschriebenen ersten Syntheseweges entstandenen Amino-desoxy-1.4 ; 3.6-dianhydro-hexite weisen in 2- oder 5-Stellung des 1.4 ; 3.6-Dianhydro-hexit-Ringsystems jeweils eine freie Hydroxylgruppe auf. Diese freie Hydroxylgruppe wird mit Salpetersäure, Nitriersäure oder mit einem Gemisch aus Salpetersäure und Eisessig/Acetanhydrid in Gegenwart von Harnstoff zu dem entsprechenden Amino-desoxy-1.4 ; 3.6-dianhydro-hexit-mononitrat verestert, wobei anstelle des freien Amino-desoxy-1.4 ; 3.6-dianhydro-hexits zur Veresterung auch ein geeignetes Säureadditionssalz, beispielsweise das entsprechende Salz der Methansulfonsäure, Salpetersäure oder Schwefelsäure eingesetzt werden kann.

Das so entstandene Mononitrat wird anschließend mit einem reaktiven Purinderivat gegebenenfalls in Gegenwart einer Hilfsbase zu der gewünschten erfindungsgemäßen Verbindung kondensiert. Geeignete Purinderivate sind die N-(ω-Halogenalkyl)-, insbesondere die N-(ω-Bromalkyl)-, N-(Epoxyalkyl)- und N-(ω-Acyloxy-alkyl)-Derivate der N',N''-dialkylsubstituierten Xanthine und die entsprechenden 9-N-Derivate des Adenins. Als ω-Acyloxyalkyl-Derivate eignen sich besonders die ω-Methansulfonyloxy- oder -p-Toluolsulfonyloxy-alkyl-Derivate.

Zur Herstellung derjenigen erfindungsgemäßen Verbindungen, bei denen das Isohexid-Ringsystem durch eine geradkettige, unsubstituierte Alkylgruppe mit einem der Ring-Stickstoffatome des Purinderivats verbunden ist, wird das entsprechende Amino-desoxy-1.4 ; 3.6-dianhydro-hexit-nitrat vorzugsweise mit einem N-(ω-Brom- oder ω-Methansulfonyloxy-alkyl)- theophyllin, -theobromin, -dialkylxanthin oder -adenin umgesetzt, also beispielsweise mit 7-(2-Bromäthyl)- theophyllin, 7-(3-Brompropyl)-theophyllin, 7-(3-Methansulfonyloxypropyl)-theophyllin, 7-(4-Brombutyl)-theophyllin, 7-(5-Brompentyl)-theophyllin, 7-(6-Bromhexyl)-theophyllin, 1-(3-Brompropyl)-theobromin, 3-Isobutyl-1-methyl-7-(3-Brompropyl)-xanthin, 9-(2-Bromäthyl)-adenin, 9-(Brompropyl)-adenin, 9-(4-Brombutyl)-adenin, 9-(5-Brompentyl)- adenin oder 9-(6-Bromhexyl)-adenin umgesetzt.

Zur Herstellung derjenigen erfindungsgemäßen Verbindungen, bei denen die « Brücke » zwischen dem Amino-isohexid-Ringsystem und dem Purinderivat durch eine Hydroxyalkylgruppe gebildet wird, wird das entsprechende Amino-desoxy-1.4 ; 3.6-dianhydro-hexit-nitrat mit einem Epoxyalkyl-Derivat der gewünschten Purinbase, beispielsweise mit 7-(2.3-Epoxypropyl)-theophyllin, umgesetzt.

Zur Herstellung derjenigen erfindungsgemäßen Verbindungen, bei denen die « Brücke » zwischen dem Aminoisohexid und dem Purinderivat eine Alkylengruppe ist, kann man nach einem weiteren erfindungsgemäßen Verfahren die beiden letzten Reaktionsschritte in der Weise vertauschen, daß man zunächst den entsprechenden Aminoisohexid in der zuvor beschriebenen Weise mit einem reaktiven Purinderivat, wie z. B. ω-Brom- oder ω-Methansulfonyloxy-alkylderivat des Theophyllins, Theobromins, N, N-dialkylierter Xanthine oder des Adenins, zum entsprechenden Purinylalkylaminoisohexid umsetzt und diesen in der zuvor beschriebenen Weise mit Salpetersäure verestert.

Zur Vermeidung der Disubstitution am Stickstoff des Aminoisohexids kann der Aminstickstoff des Aminoisohexids vor der Umsetzung mit dem reaktiven Purinderivat mit einer geeigneten Schutzgruppe, z. B. dem Benzylrest, versehen werden, die nach der Umsetzung mit dem reaktiven Purinderivat leicht wieder abgespalten werden kann, z. B. durch Hydrierung in Gegenwart eines der üblichen Edelmetallkatalysatoren.

Ein weiteres geeignetes Verfahren zur Herstellung derjenigen erfindungsgemäßen Verbindungen, bei denen die « Brücke » zwischen dem Aminoisohexid und dem Purinderivat eine Alkylengruppe ist, besteht darin, daß man den entsprechenden Aminoisohexid zusammen mit einem ω-Acylalkyl- bzw. ω-Oxoalkyl-xanthin- oder -adenin-Derivat in an sich bekannter Weise einer reduktiven Kondensation in Gegenwart von Wasserstoff und einem geeigneten Edelmetall- oder Edelmetallsulfid-Katalysator, gegebenenfalls in Gegenwart eines Lösungsmittels, unterwirft. Im Falle der ω-Oxoalkylxanthin- oder -adenin-Derivate, beispielsweise des 7-(3-Oxopropyl)-theophyllins, entstehen die entsprechenden Purinylalkylamino-isohexid-Derivate mit unverzweiger Alkylenbrücke, z. B. 3-Theophyllin-7-yl-propylamino-isohexid, deren freie Hydroxygruppe im folgenden Reaktionsschritt in der zuvor schon beschriebenen Weise mit Salpetersäure, Nitriersäure oder Salpetersäure/Acetanhydrid/Essigsäure zum erfindungsgemäßen Endprodukt, z. B. 3-Theophyllin-7-yl-propylamino-isohexic-nitrat, verestert wird.

Im Falle der ω-Acylalkyl-xanthin- oder -adenin-Derivate, z. B. des 7-(2-Acetyläthyl)-theophyllins oder 7-(4-Acetyl-butyl)-theophyllins, entstehen bei der reduktiven Kondensation mit dem Aminoisohexid und nachfolgender Veresterung mit Salpetersäure, Nitriersäure oder Salpetersäure/Acetanhydrid/Essigsäure diejenigen erfindungsgemäßen ω-Purinyl-alkylamino-isohexid-nitrate, bei denen die Alkylenbrücke verzweigt ist, wie z. B. bei dem 3-Theophyllin-7-yl-1-methyl-propylamino-isohexid-nitrat. Die so erhaltenen

erfindungsgemäßen Verbindungen mit verzweigter Alkylenbrücke besitzen ein zusätzliches Asymmetriezentrum, so daß jeweils zwei Diastereomere bei der reduktiven Kondensation anfallen. Diese können entweder sofort oder besser nach der Veresterung zu den entsprechenden Salpetersäureestern mit Hilfe der üblichen Trennmethoden, z. B. durch Säulenchromatographie, Flüssig-Flüssig-Verteilung oder fraktionierte Kristallisation, in die beiden isomeren Endprodukte, z. B. (+)-5-(3-Theophyllin-7-yl-1-methylpropylamino)-5-desoxy-1.4 ; 3.6-dianhydro-L-idit-2-nitrat und in das entsprechende (−)-Isomere, aufgetrennt werden.

Der bisher beschriebene erste erfindungsgemäße, Weg zur Herstellung der erfindungsgemäßen Verbindungen, bei dem der entsprechende Isohexid mit einem Sulfonsäurechlorid, vorzugsweise mit Methansulfonsäurechlorid oder Toluol-sulfonsäurechlorid, in den entsprechenden Monoacyl-1.4 ; 3.6-dianhydro-hexit übergeführt wird, hat den Nachteil, daß bei der Acylierung nicht nur das entsprechende 5-O-Acyl-Derivat bzw. 2-O-Acyl-Derivat entsteht, sondern gleichzeitig auch das 2,5-Diacyl-Derivat, so daß bei den Isoidid- und Isomannid-Derivaten jeweils die Monoacyl-Verbindung vom Diacylat abgetrennt werden muß, während beim Isosorbid, bei dem zwei stereoisomere Monoacyl-Derivate neben dem Diacylat entstehen, das gewünschte Acylat aus dem Gemisch der drei Acyl-Derivate isoliert werden muß. Die Auftrennung des Acylatgemisches erfolgt entweder durch fraktionierte Kristallisation, fraktionierte Extraktion oder mit Hilfe anderer an sich bekannter Methoden.

Die mühsame und aufwendige Auftrennung des Acylat-Gemisches entfällt jedoch bei Anwendung des erfindungsgemäßen zweiten Syntheseweges zu den 5-Purinyl-alkylamino-isoidid-Derivaten dadurch, daß 1.4 ; 3.6-Dianhydro-D-glucit mit einem Überschuß an Sulfonsäurechlorid, vorzugsweise Methansulfonylchlorid oder Toluolsulfonylchlorid, in Pyridin oder Chloroform/Triäthylamin quantitativ zum entsprechenden 1.4 ; 3.6-Dianhydro-D-glucit-2.5-diacylat umgesetzt wird.

Das Diacylat wird dann unter entsprechenden Bedingungen, wie bei dem ersten Syntheseweg beschrieben, der Ammonolyse unterworfen, wobei neben einer geringen, in der wäßrigen Phase verbleibenden Menge an 2.5-Diamino-2.5-didesoxy-1.4 ; 3.6-dianhydro-D-glucit ein Gemisch aus 5-Amino-5-desoxy-1.4 ; 3.6-dianhydro-L-idit und 5-Amino-5-desoxy-1.4 ; 3.6-dianhydro-L-idit-2-methansulfonat im Verhältnis 1 : 4 entsteht.

Dieses 1 : 4-Gemisch wird dann einer alkalischen oder sauren Hydrolyse unterworfen, und der entsprechende 5-Amino-5-desoxy-1.4 ; 3.6-dianhydro-L-idit wird anschließend, wie beim ersten Syntheseweg beschrieben, zum entsprechenden Mononitrat verestert und mit dem gewünschten Purinderivat kondensiert oder erst mit dem gewünschten Purinderivat kondensiert und anschließend zum Mononitrat verestert.

Wie sich aus der Tatsache, daß die Ammonolyse des 2.5-Diacylats überwiegend zum 5-Amino-2-acylat führt, ergibt, ist die nucleophile Substitution der 2-exo-Acylatgruppe im Isosorbid-diacylat sterisch gehindert. Der Grad der sterischen Hinderung ist temperaturabhängig. Um aus dem entsprechenden Diacylat möglichst quantitativ das 5-Amino-2-acylat zu erhalten, arbeitet man deshalb erfindungsgemäß vorzugsweise bei einer Temperatur von 80 bis 110 °C, da bei Temperaturen über 110 °C auch die 2-exo-Acylgruppe, wenn auch in geringem Ausmaß, durch Ammoniak oder von dem eingesetzten Monoalkylamin angegriffen wird. Als Lösungsvermittler kann der wäßrigen Ammoniaklösung oder dem Monoalkylamin ein Alkohol, vorzugsweise Äthanol, zugesetzt werden.

Bei einem weiteren erfindungsgemäßen Weg zur Herstellung der erfindungsgemäßen Verbindungen macht man sich die überraschend aufgefundene Tatsache zunutze, daß 1.4 ; 3.6-Dianhydro-D-glucit-2.5-diacylate (Dimesylat oder Ditosylat) von Natriumbenzoat in einem geeigneten Lösungsmittel, vorzugsweise einem dipolarem aprotischen Lösungsmittel, beispielsweise in wasserfreiem Dimethylformamid, Dimethylsulfoxid oder Diäthern des Äthylenglykols, bei Temperaturen von 100 bis 180 °C, vorzugsweise 120 bis 150 °C, selektiv am $C^5$-Atom angegriffen werden, so daß unter Konfigurationsumkehr in hoher Ausbeute 1.4 ; 3.6-Dianhydro-L-idit-2-methansulfonat-5-benzoat bzw. 1.4 ; 3.6-Dianhydro-L-idit-2-toluolsulfonat-5-benzoat entsteht. Dieses Produkt wird nun wieder der Ammonolyse mit 25 %iger Ammoniaklösung oder der Aminolyse unter erhöhtem Druck und erhöhter Temperatur unterworfen, wobei der Benzoesäureester hydrolytisch abgespalten wird, und zwar überraschenderweise unter Beibehaltung der Konfiguration am $C^5$-Atom, während der Acylatrest am $C^2$-Atom unter Konfigurationsumkehr durch die Amino- bzw. Alkylaminogruppe zum entsprechenden 5-Amino-5-desoxy-1.4 ; 3.6-dianhydro-D-glucit bzw. 5-Alkylamino-5-desoxy-1.4 ; 3.6-dianhydro-D-glucit substituiert wird.

Da das zuletzt genannte elegante Verfahren mit einer zweimaligen selektiven Konfigurationsumkehr verbunden ist, ist die Konfiguration des Endproduktes bezüglich seiner Substituenten identisch mit der Konfiguration der Ausgangsverbindung ; aus dem Isosorbid-disulfonat entsteht wieder ein Isosorbid-Derivat, nämlich 5-Amino- bzw. 5-Alkylamino-5-desoxy-1.4 ; 3.6-dianhydro-D-glucit.

Die so gewonnenen 5-Amino-5-desoxy-1.4 ; 3.6-dianhydro-D-glucit-Derivate können anschließend-1.4 ; 3.6-dianhydro-D-glucit-Derivate können anschließend in analoger Weise auf den zuvor beschriebenen Reaktionswegen mit reaktiven Purin-Derivaten zu den erfindungsgemäßen 5-(ω-Purinyl-alkylamino)-5-desoxy-1.5 ; 3.6-dianhydro-D-glucit-Derivaten kondensiert werden, wobei die Veresterung mit Salpetersäure je nach Verfahrensvariante vor oder nach Kondensation erfolgen kann.

Um die erfindungsgemäßen Verbindungen in ihre physiologisch unbedenklichen Salze überzuführen, können anorganische Säuren und Mineralsäuren wie Halogenwasserstoffsäuren, Schwefelsäure oder Phosphorsäuren sowie organische Säuren wie Carbon- und Sulfonsäuren, beispielsweise Malon-Bernstein-, Milch-, Wein-, Äpfel-, Benzoe-, Salicyl-, Citronen-, Ascorbin-, Nicotin- oder p-Toluolsulfonsäure verwendet werden. Die freien Basen können aus den Säureadditionssalzen wieder durch Behandlung mit starken Basen, beispielsweise Natrium- oder Kaliumhydroxid, freigesetzt werden.

Gegenstand der Erfindung sind ferner pharmazeutische Zubereitungen, die neben üblichen Träger- und Zusatzstoffen mindestens eine der erfindungsgemäßen Verbindungen oder ihrer physiologisch unbedenklichen Salze enthalten. Diese Zubereitungen können als Arzneimittel in der Human- oder Veterinärmedizin verwendet werden. Üblich Trägerstoffe sind beispielsweise Wasser, pflanzliche Öle, Polyäthylenglykole, Glycerinester, Gelatine, Kohlenhydrate wie Laktose oder Stärke, Magnesiumstearat, Talk, Vaseline. Übliche Zusatzstoffe sind beispielsweise Konservierungs-, Stabilisierungs-, Gleit-, Netzmittel, Emulgatoren, physiologisch unbedenkliche Salze, Puffersubstanzen, Farb-, Geschmacks- und Aromastoffe. Die Auswahl der Träger- und Zusatzstoffe hängt davon ab, ob die erfindungsgemäßen Verbindungen enteral, parenteral oder topikal appliziert werden sollen.

Die erfindungsgemäßen Verbindungen können auch im Gemisch mit anderen Wirkstoffen, beispielsweise Vitaminen oder bekannten, im Handel befindlichen Herz-Kreislauf-Mitteln, insbesondere auch mit β-Rezeptorenblockern, verabreicht werden.

Beispiel für eine pharmazeutische Zubereitung :

Für die Herstellung von Tabletten von je 100 mg Einzelgewicht, die je 5 mg Wirkstoff enthalten, benötigt man z. B.

I. 5 g 5-(3-Theophyllin-7-yl-propyl)-amino-5-desoxy-1.4 ; 3.6-dianhydro-L-idit-2-nitrat-Hydrochlorid
II. 54 g mikrokristalline Cellulose
III. 20 g Milchzucker
IV. 20 g Maisstärke
V. 0,5 g kolloidale Kieselsäure
VI. 0,5 g Magnesiumstearat

Die Substanzen I-IV werden trocken 10 Minuten lang gemischt, anschließend wird das Gemisch der Substanzen V und VI zugegeben, man mischt weitere 10 Minuten und verpreßt das so erhaltene Pulver auf einer Tablettiermaschine zu Tabletten von 100 mg Einzelgewicht.

Jede der in den nachfolgenden Beispielen genannten erfindungsgemäßen Verbindungen und Zwischenprodukte stellt ein zur Herstellung pharmazeutischer Zubereitungen besonders geeignetes Mittel dar.

Die in den Beispielen enthaltenen Abkürzungen haben folgende Bedeutungen :

Schmp. = Schmelzpunkt (unkorrigiert)
(Z) = Zersetzung
d = Dichte
$[a]_D^{25}$ = optische Drehung bei 25 °C, Natrium-D-Linie.

Hinter den optischen Drehwerten sind die Konzentrationen der gemessenen Lösungen angegeben, wobei c 2 beispielsweise eine Konzentration von 2 g/100 ml Lösung bedeutet ; das Lösungsmittel ist jeweils gesondert angegeben.

Alle Temperaturen sind in °C angegeben.

Beispiel 1

5-(3-Theophyllin-7-ylpropyl)-amino-5-desoxy-1.4 ; 3.6-dianhydro-L-idit-2-nitrat :

a) 1.4 ; 3.6-Dianhydro-D-glucit-2-methansulfonat, -5-methansulfonat und -2.5-dimethansulfonat :

Zur Lösung von 4.82 kg (33 Mol) 1.4 ; 3.6-Dianhydro-D-glucit in 24 Liter Pyridin tropft man unter Feuchtigkeitsausschluß, Rühren und Kühlen auf − 15° bis − 20° innerhalb mehrerer Stunden 3.1 Liter (40 Mol) Methansulfonsäurechlorid. Anschließend rührt man 15 Stunden ohne Kühlung weiter. Man destilliert im Vak. das Pyridin ab, gibt zum öligen Rückstand 15 Liter Wasser, kocht auf und läßt abkühlen. Absaugen, Waschen mit 4 Litern Wasser und Trocknen des kristallinen Niederschlags ergibt 2.22 kg (7.34 Mol) 1.4 ; 3.6-Dianhydro-D-glucit-2.5-dimethansulfonat. Das Filtrat wird unter Rühren und Wasserkühlung mit ca. 1.5 kg Natriumhydroxid neutralisiert und bei ca. 70° im Vakuum bis zur Trockne eingedampft. Der Trockenrückstand wird mit insgesamt 30 Litern Chloroform kontinuierlich heiß extrahiert und der Extrakt heiß filtriert. Man läßt den Extrakt 15 Stunden bei 20° stehen, saugt den kristallinen Niederschlag ab, wäscht ihn zweimal mit je 2 Litern Chloroform, trocknet und erhält 2.3 kg (10.26 Mol) 1.4 ; 3.6-Dianhydro-D-glucit-5-methansulfonat. Die vereinigten Filtrate werden im Vakuum eingedampft und der Rückstand heiß in 22 Litern Aethanol gelöst. Man läßt 15 Stunden bei 20° stehen, saugt den kristallinen Niederschlag ab, wäscht ihn zweimal mit je 3 Litern Aethanol, trocknet und erhält 0.65 kg (2.90 Mol) 1.4 ; 3.6-Dianhydro-D-glucit-2-methansulfonat. Eindampfen der Filtrate ergibt 2.21 kg (9.85 Mol) eines Gemisches der beiden

isomeren Mono-methansulfonate, das je nach Bedarf durch Wiederholung der abwechselnden Kristallisationen aus Chloroform und Aethanol weiter aufgetrennt werden kann, bzw. durch Veresterung mit Methansulfonsäurechlorid in Pyridin vollständig in 1.4 ; 3.6-Dianhydro-D-glucit-2.5-dimethansulfonat übergeführt wird.

Analytische Mengen der Methansulfonate ergeben nach Umkristallisation korrekte Elementaranalysen und die in Tabelle 1 aufgeführten Schmelzpunkte und optischen Drehungen :

Tabelle 1

| 1.4;3.6-Dianhydro-D-glucit- | Umkristallisiert aus | Schmp. [°C] | $[\alpha]_D^{25}$ |
|---|---|---|---|
| -2-methansulfonat | Chloroform | 135-138.5 | 62.5 (c 2; Aceton) |
| -5-methansulfonat | Chloroform | 123-124 | 75.9 (c 2;Methanol) |
| 2.5-dimethansulfonat | Aethanol / Aceton | 127-128 | 74 (c 2;Aceton) |

Anmerkung :

Setzt man 1.4 ; 3.6-Dianhydro-D-glucit mit der 2 bis 2.5-fach molaren Menge Methansulfonsäurechlorid unter den gleichen Reaktionsbedingungen um, erhält man das 1.4 ; 3.6-Dianhydro-D-glucit-2.5-dimethansulfonat in nahezu quantitativer Ausbeute.

b) 5-Amino-5-desoxy-1.4 ; 3.6-dianhydro-L-idit :

Dieses Zwischenprodukt kann auf 2 Wegen erhalten werden :

Verfahren 1

Herstellung durch Ammonolyse von 1.4 ; 3.6-Dianhydro-D-glucit-5-methansulfonat. Das Gemisch aus 448 g (2 Mol) 1.4 ; 3.6-Dianhydro-D-glucit-5-methansulfonat und 1.5 Liter 25 %igem wäßrigen Ammoniak (20 Mol) wird im geschlossenen Stahlautoklaven 24 Stunden bei 130° gerührt. Danach ist die Umsetzung quantitativ. Man dampft unter vermindertem Druck ein und trocknet azeotrop durch aufeinanderfolgende Zugabe und erneutes Eindampfen von je 1 Liter Aethanol und Chloroform nach. Der ölige Rückstand wird unter Erwärmen in 500 ml Aethanol gelöst und mit Isopropanol auf 2 Liter verdünnt. Beim Erkalten kristallisieren 311 g (1.3 Mol) 5-Amino-5-desoxy-1.4 ; 3.6-dianhydro-L-idit als methansulfonsaures Salz aus. Weitere 100 g (0.4 Mol) kristallines Reinprodukt fallen nach Behandeln der Mutterlauge mit 30 g Aktivkohle und Konzentrieren des Filtrats aus. Zur Analyse kristallisiert man aus Aethanol/Chloroform um.

Schmp. 151-4° ; $[\alpha]_D^{25}$ 27.6 (c 1 ; Wasser)

Elementaranalyse : $C_6H_{11}NO_3 \times CH_3SO_3H$ (241.27)

Ber. : C (34.83), H (6.27), N (5.81)
Gef. : C (34.71), H (6.45), N (5.36)

Ein kleiner Teil des Produkts wird in die freie Base übergeführt und aus Chloroform/Aether umkristallisiert. Schmp. 103-104° ; $[\alpha]_D^{25}$ 31.6 (c 2 ; Wasser)

Verfahren 2

Ammonolyse von 1.4 ; 3.6-Dianhydro-D-glucit-2.5-dimethansulfonat gefolgt von alkalischer Hydrolyse des erhaltenen 5-Amino-5-desoxy-1.4 ; 3.6-dianhydro-L-idit-2-methansulfonats.

Das Gemisch aus 302 g (1 Mol) 1.4 ; 3.6-Dianhydro-D-glucit-2.5-dimethansulfonat, 750 ml 25 %igem wäßrigen Ammoniak (10 Mol) und 750 ml Aethanol wird im verschlossenen Stahlautoklaven 4 Tage bei 100° gerührt. Nach dem Abkühlen versetzt man mit 1 Liter Wasser und saugt vom auskristallisierten nicht umgesetzten Dimethansulfonat (106 g = 0.35 Mol) ab. Das Filtrat wird zur Entfernung von Ammoniak mit 104 g (1.3 Mol). Natriumhydrogencarbonat versetzt und unter vermindertem Druck eingedampft. Man löst in 5 Liter Wasser und extrahiert mit 500 ml Chloroform Eliminierungsprodukte heraus. Die wäßrige Phase wird 48 Stunden lang im Rotationsperforator (Normag) kontinuierlich mit Chloroform extrahiert. In der Wasserphase verbleibt als Nebenprodukt entstandener 2.5-Diamino-2.5-didesoxy-1.4 ; 3.6-dianhydro-D-glucit. Der Chloroformextrakt ergibt nach dem Trocknen über wasserfreiem Natriumsulfat, Filtrieren und Eindampfen 105 g (ca. 0.55 Mol) eines 1 : 4 Gemisches aus 5-Amino-5-desoxy-1.4 ; 3.6-dianhydro-L-idit und 5-Amino-5-desoxy-1.4 ; 3.6-dianhydro-L-idit-2-methansulfonat.

Zur Charakterisierung des letzteren Produkts wird ein kleiner Teil des Gemisches in Chloroform gelöst, 2 mal mit Wasser gewaschen, die Chloroformphase eingedampft, in das methansulfonsaure Salz übergeführt und 2 mal aus Aethanol umkristallisiert.

Schmp. 213-5° ; $[\alpha]_D^{25}$ 39.0 (c 0.50 ; Wasser)

Elementaranalyse : $C_7H_{13}NO_5S \times CH_3SO_3H$ (319.37)

Ber. : C (30.09), H (5.37), N (4.39), S (20.08)
Gef. : C (30.13), H (5.49), N (4.25), S (20.6 )

Das oben erhaltene Gemisch wird in zu einer Lösung von 60 g (1.5 Mol) Natriumhydroxid in 1.5 Liter Wasser gegeben und 24 Stunden unter Rückfluß gekocht. Nach dem Abkühlen stellt man durch Zugabe von konz. Salzsäure auf pH = 10 ein, filtriert und dampft unter vermindertem Druck ein, trocknet aceotrop mit n-Butanol nach, erwärmt den Rückstand mit 500 ml n-Butanol und filtriert von anorganischen Salzen ab. Die butanolische Lösung wird eingedampft, der Rückstand in 200 ml Isopropanol gelöst und mit 34 g (0.35 Mol) Methansulfonsäure versetzt. Es kristallisieren 80 g (0.33 Mol) 5-Amino-5-desoxy-1.4 ; 3.6-dianhydro-L-idit in Form des methansulfonsauren Salzes aus Schmp. 150-2°. Ausbeute bezogen auf umgesetztes 1.4 ; 3.6-Dianhydro-D-glucit-2.5-dimethansulfonat : 50 %.

c) 5-Amino-5-desoxy-1.4 ; 3.6-dianhydro-L-idit-2-nitrat :

241 g (1 Mol) 5-Amino-5-desoxy-1.4 ; 3.6-dianhydro L-idit-Hydrogenmethansulfonat (bzw. 145 g der freien Base) werden in 50 ml Wasser gelöst und unter Kühlung tropfenweise mit 50 ml konz. Schwefelsäure (d = 1.84 ; 0.54 Mol) versetzt (Lösung A).

Zu 200 ml 96 %iger Salpetersäure (d = 1.5 ; 4.5 Mol) tropft man unter Rühren und Kühlen auf – 15° die Lösung von 14 g (0.23 Mol) Harnstoff in 300 ml konz. Schwefelsäure (d = 1.84 ; 5.4 Mol). Anschließend tropft man bei – 15° die Lösung A innerhalb 3-4 Stdn. zu und rührt noch 2 Stdn. bei dieser Temperatur. Die Reaktionsmischung wird langsam in 1.5 Liter Wasser eingerührt. Unter Kühlen neutralisiert man durch langsame Zugabe einer Lösung von 630 g (15.75 Mol) Natrium-hydroxid (bzw. 590 g NaOH, falls zuvor die freie Base eingesetzt wurde) in 2 Litern Wasser und filtriert. Das Filtrat wird 16 Stdn. im 5 Liter-Rotationsperforator kontinuierlich mit Chloroform extrahiert. Aus dem Chloroformextrakt erhält man nach Trocknen über wasserfreiem Natriumsulfat, Filtrieren und Eindampfen unter vermindertem Druck 158 g (0.83 Mol) 5-Amino-5-desoxy-1.4 ; 3.6-Dianhydro-L-idit-2-nitrat als langsam kristallisierendes Oel. Zur Charakterisierung überführt man einen kleinen Anteil in das Hydrochlorid und kristallisiert aus Isopropanol um.

Schmp. 201-208° (Z) ; $[\alpha]_D^{25}$ 54.1 (c 2 ; Wasser)

Elementaranalyse : $C_6H_{10}N_2O_5 \times HCl$ (226.62)

Ber. : C (31.80), H (4.89), N (12.36), Cl (15.64).
Gef. : C (31.78), H (4.92), N (12.20), Cl (15.6 ).

d) 5-(3-Theophyllin-7-ylpropyl)-amino-5-desoxy-1.4 ; 3.6-dianhydro-L-idit-2-nitrat :

Im 1-Liter-Soxhlet-Extraktor wird das Gemisch aus 93 g (0.49 Mol) 5-Amino-5-desoxy-1.4 ; 3.6-dianhydro-L-idit-2-nitrat und 500 ml Aethanol vorgelegt und die Extraktionshülse mit 66 g (0.22 Mol) 7-(3-Brompropyl)-theophyllin [Herstellung z. B. nach H. Priewe u. A. Poljak, Chem. Ber. 90, 1651-5 (1957)] beschickt. Unter Rühren erhitzt man 18 Stdn. unter Rückfluß, wobei der Hülseninhalt nach ca. 3 Stdn. vollständig in die Reaktionsmischung extrahiert worden ist. Nach dem Abkühlen gibt man 400 ml Chloroform zu, saugt von ausgefallenem überschüssigen 5-Amino-5-desoxy-1.4 ; 3.6-dianhydro-L-idit 2-nitrat-Hydrobromid ab und wäscht mit je 200 ml Aethanol und Chloroform nach. Das Filtrat wird nach dem Eindampfen unter vermindertem Druck in 400 ml Chloroform gelöst, nacheinander 2 mal mit je 100 ml Wasser und mit 100 ml 1-molarer Natronlauge gewaschen, wobei restliches 5-Amino-5-desoxy-1.4 ; 3.6-dianhydro-L-idit-2-nitrat entfernt wird, und anschließend 9 mal mit je 100 ml 0.2-molarer Salzsäure extrahiert. Die verbleibende Chloroformphase und die Salzsäurephase Nr. 9 enthalten Disubstitutionsprodukt und werden verworfen. Die Salzsäurephasen Nr. 1-8 werden unter vermindertem Druck eingedampft und aus Aethanol/Wasser umkristallisiert. Man erhält 61.4 g (137 mmol) 5-(3-Theophyllin-7-ylpropyl)-amino-5-desoxy-1.4 ; 3.6-dianhydro-L-idit-2-nitrat in Form des Hydrochlorids.

Schmp. 207-8° (Z) ; $[\alpha]_D^{25}$ 29.4 (c 0.5 ; Wasser)
Elementaranalyse : $C_{16}H_{22}N_6O_7 \times HCl$ (446.85)
Ber. : C (43.01), H (5.19), N (18.81), Cl (7.93)
Gef. : C (42.98), H (5.35), N (18.40), Cl (8.5 )

## Beispiel 2

5-(2-Theophyllin-7-yläthyl)-amino-5-desoxy-1.4 ; 3.6-dianhydro-L-idit-2-nitrat.

Herstellung analog Beispiel 1d) durch Umsetzung von überschüssigem 5-Amino-5-desoxy-1.4 ; 3.6-dianhydro-L-idit-2-nitrat mit 7-(2-Bromäthyl)-theophyllin. Das Produkt wird als Hydrochlorid isoliert und aus Aethanol umkristallisiert. Ausbeute : 47 %

Schmp. 166° (Z) ; $[\alpha]_D^{25}$ 36.8 (c 0.5 ; Wasser)
Elementaranalyse : $C_{15}H_{20}N_6O_7 \times HCl$ (432.83)
Ber. : C (41.63), H (4.89), N (19.42), Cl (8.19)
Gef. : C (41.30), H (5.01), N (19.21), Cl (8.1 )

Beispiel 3

5-(4-Theophyllin-7-ylbutyl)-amino-5-desoxy-1.4 ; 3.6-dianhydro-L-idit-2-nitrat.

Herstellung analog Beispiel 1d) durch Umsetzung von überschüssigem 5-Amino-5-desoxy-1.4 ; 3.6-dianhydro-L-idit-2-nitrat mit 7-(4-Brombutyl)-theophyllin. Das Produkt wird als Hydrochlorid isoliert und aus Aethanol/Isopropanol umkristallisiert. Ausbeute : 41 %
Schmp. 183° (Z) ; $[\alpha]_D^{25}$ 32.1 (c 0.5 ; Wasser)
Elementaranalyse : $C_{17}H_{24}N_6O_7 \times HCl$ (460.88)
Ber. : C (44.30), H (5.47), N (18.23), Cl (7.69)
Gef. : C (43.94), H (5.49), N (17.87), Cl (8.0 )

Beispiel 4

5-(5-Theophyllin-7-ylpentyl)-amino-5-desoxy-1.4 ; 3.6-dianhydro-L-idit-2-nitrat.

Herstellung analog Beispiel 1d) durch Umsetzung von überschüssigem 5-Amino-5-desoxy-1.4 ; 3.6-dianhydro-L-idit-2-nitrat mit 7-(5-Brompentyl)-theophyllin. Das Produkt wird als Hydrochlorid isoliert und aus Aethanol/Isopropanol umkristallisiert. Ausbeute : 39 %
Schmp. 191° (Z) ; $[\alpha]_D^{25}$ 31.7 (c. 0.5 ; Wasser)
Elementaranalyse : $C_{18}H_{26}N_6O_7 \times HCl$ (474.91)
Ber. : C (45.52), H (5.73), N (17.40), Cl (7.46)
Gef. : C (45.86), H (5.86), N (17.67), Cl (7.6 )

Beispiel 5

5-(6-Theophyllin-7-ylhexyl)-amino-5-desoxy-1.4 ; 3.6-dianhydro-L-idit-2-nitrat.

Herstellung analog Beispiel 1d) durch Umsetzung von überschüssigem 5-Amino-5-desoxy-1.4 ; 3.6-dianhydro-L-idit-2-nitrat mit 7-(6-Bromhexyl)-theophyllin. Das Produkt wird als Hydrochlorid isoliert und aus Aethanol/Isopropanol umkristallisiert. Ausbeute : 42 %
Schmp. 152-4° (Z) ; $[\alpha]_D^{25}$ 33.5 (c 1 ; chloroform)
Elementaranalyse : $C_{19}H_{28}N_6O_7 \times HCl$ (488.93)
Ber. : C (46.68), H (5.98), N (17.19), Cl (7.25)
Gef. : C (46.80), H (6.09), N (17.09), Cl (7.6 )

Beispiel 6

5-(2-Hydroxy-3-theophyllin-7-ylpropyl)-amino-5-desoxy-1.4 ; 3.6-dianhydro-L-idit-2-nitrat.

Die Mischung aus 1.9 g (10 mmol) 5-Amino-5-desoxy-1.4 ; 3.6-dianhydro-L-idit, 20 ml 96 %igem Aethanol und 1.65 g (7 mmol) 7-(2.3-Epoxypropyl)-theophyllin [Herstellung z. B. nach C.A. 57, P 13 777 f (1962)] wird 20 Stdn. unter Rückfluß gekocht und unter vermindertem Druck eingedampft. Der Rückstand wird über eine Säule mit 340 g Kieselgel (70-230 mesh, Woelm) und Methanol/Chloroform 8/2 als Elutionsmittel chromatographisch aufgetrennt. Die das Reaktionsprodukt enthaltenden Fraktionen (Rf = 0.54 ; Kieselgel-Fertigplatten Merck F254, Chloroform/Methanol 8/2) werden vereinigt und unter vermindertem Druck eingedampft. Man erhält 2.3 g (5.4 mmol) 5-(2-Hydroxy-3-theophyllin-7-ylpropyl)-amino-5-desoxy-1.4 ; 3.6-dianhydro-L-idit-2-nitrat, die in das Hydrochlorid übergeführt und aus Isopropanol/Aethanol umkristallisiert werden.
Schmp. 195-8° (Z) ; $[\alpha]_D^{25}$ 24.4 (c 1 ; Wasser)
Elementaranalyse : $C_{16}H_{22}N_6O_8 \times HCl$ (462.85)
Ber. : C (41.74), H (5.01), N (18.16), Cl (7.66)
Gef. : C (41.74), H (5.07), N (18.06), Cl (7.7 )

Beispiel 7

5-[3-(3.7-Dimethylxanthin-1-yl)-propylamino]-5-desoxy-1.4 ; 3.6-dianhydro-L-idit-2-nitrat :

a) 1-(3-Hydroxypropyl)-theobromin :

Zur Mischung aus 36 g (0.2 Mol) Theobromin, 25 ml (0.3 Mol) 3-Chlor-1-propanol und 100 ml Wasser tropft man innerhalb 2 Stdn. unter Rühren und Erhitzen zum Rückfluß die Lösung von 12 g (0.3 Mol) Natriumhydroxid in 30 ml Wasser und kocht noch 2 Stdn. nach. Nach dem Abkühlen filtriert man, dampft das Filtrat unter vermindertem Druck ein, kocht den Rückstand 2 mal mit je 100 ml Aethanol aus und filtriert heiß. Das beim Abkühlen der Aethanolextrakte auskristallisierende Rohprodukt liefert nach erneuter Umkristallisation aus Aethanol 24.3 g (0.1 Mol) 1-(3-Hydroxypropyl)-theobromin. Schmp. 139-140°

b) 1-(3-Brompropyl)-theobromin :

Zur Mischung aus 16.7 g (70 mmol) 1-(3-Hydroxypropyl)-theobromin und 100 ml Chloroform tropft man unter Rühren und Erhitzen zum Rückfluß langsam 13 ml (140 mmol) Phosphortribromid und rührt noch 4 Stdn. unter Rückfluß nach. Die abgekühlte Reaktionsmischung wird in 200 ml Eiswasser eingerührt. Nach Abtrennen der Chloroformphase extrahiert man die wäßrige Phase 3 mal mit je 50 ml Chloroform, wäscht die vereinigten Chloroformphasen mit 100 ml Wasser, trocknet über wasserfreiem Natriumsulfat, filtriert und dampft ein. Der Rückstand (18.1 g) liefert nach Umkristallisation aus Aethanol 16.2 g (54 mmol) 1-(3-Brom-propyl)-theobromin. Schmp. 142-144°

c) 5-[3-(3.7-Dimethylxanthin-1-yl)-propylamino]-5-desoxy-1.4 ; 3.6-dianhydro-L-idit-2-nitrat :

Die Mischung aus 9.0 g (30 mmol) 1-(3-Brompropyl)-theobromin, 100 ml Aethanol und 14.3 g (75 mmol) 5-Amino-5-desoxy-1.4 ; 3.6-dianhydro-L-idit-2-nitrat (Herstellung siehe Beispiel 1c) wird 24 Stdn. unter Rückfluß gekocht. Nach dem Abkühlen filtriert man als Hydrobromid ausfallendes überschüssiges 5-Amino-5-desoxy-1.4 ; 3.6-dianhydro-L-idit-2-nitrat (7.3 g ; 27 mmol) ab und dampft das Filtrat unter vermindertem Druck ein. Der Rückstand wird in 100 ml Chloroform gelöst, durch Waschen mit 5 %iger Essigsäure von restlichem Ausgangsamin befreit, anschließend mit 2 %iger Natronlauge säurefrei gewaschen und nach dem Trocknen über wasserfreiem Natriumsulfat unter vermindertem Druck eingedampft. Man erhält 9.56 g (23.3 mmol) Rohprodukt, die mit 24 mmol äthanolischer Salzsäure in das Hydrochlorid übergeführt werden. Nach dem Eindampfen kristallisiert man aus Aethanol um und erhält 6.9 g (15.1 mmol) 5-[3-(3.7-Dimethylxanthin-1-yl)-propylamino]-5-desoxy-1.4 ; 3.6-dianhydro-L-idit-2-nitrat-Hydrochlorid mit 1/2 Mol Kristallwasser.

Schmp. 176-180 °C (Z) ; $[\alpha]_D^{25}$ 28.9 (c. 0.4 ; Wasser)

Elementaranalyse : $C_{16}H_{22}N_6O_7 \times HCl \times 1/2\ H_2O$ (455.86)

| | | | | |
|---|---|---|---|---|
| Ber. : | C (42.16), | H (5.31), | N (18.44), | Cl (7.78) |
| Gef. : | C (42.16), | H (5.19), | N (18.32), | Cl (8.2 ) |

## Beispiel 8

5-[3-(3-Isobutyl-1-methylxanthin-7-yl)-propylaminol]-5-desoxy-1.4 ; 3.6-dianhydro-L-idit-2-nitrat ;

a) 3-Isobutyl-1-methyl-7-(3-hydroxypropyl)-xanthin :

Zu dem Gemisch aus 10 g (45 mmol) 3-Isobutyl-1-methyl-xanthin, 40 ml Wasser und 5.4 ml (65 mmol) 3-Chlor-1-propanol tropft man innerhalb 2 Stdn. die Lösung von 2.6 g (65 mmol) Natriumhydroxid in 10 ml Wasser unter Rühren und Erhitzen zum Rückfluß und kocht noch 2 Stdn. nach. Man läßt über Nacht bei Raumtemperatur stehen, saugt das auskristallisierte Produkt ab und kristallisiert aus Aethanol um. Man erhält 5.9 g (21 mmol) 3-Isobutyl-1-methyl-7-(3-hydroxypropyl)-xanthin.

Schmp. 135-7°

b) 3-Isobutyl-1-methyl-7-(3-brompropyl)-xanthin :

Zur Mischung aus 5.1 g (18 mmol) 3-Isobutyl-1-methyl-7-(3-hydroxypropyl)-xanthin und 80 ml Chloroform tropft man unter Rühren und Erhitzen zum Rückfluß langsam 3.7 ml (40 mmol) Phosphortribromid und kocht noch 3 Stdn. Die abgekühlte Mischung wird in 100 ml Eiswasser eingerührt, die Chloroformphase abgetrennt und die wäßrige Phase 3 mal mit je 30 ml Chloroform extrahiert. Die vereinigten Chloroformphasen ergeben nach dem Waschen mit 50 ml Wasser, Trocknen über wasserfreiem Natriumsulfat, Filtrieren und Eindampfen unter vermindertem Druck 6.0 g (17.5 mmol) 3-Isobutyl-1-methyl-7-(3-brompropyl)-xanthin als farbloses Oel, das ohne weitere Reinigung in der folgenden Reaktionsstufe eingesetzt wird.

c) 5-[3-(3-Isobutyl-1-methylxanthin-7-yl)-propylamino)-5-desoxy-1.4 ; 3.6-dianhydro-L-idit-2-nitrat :

5 g (14.6 mmol) des zuvor beschriebenen 3-Isobutyl-1-methyl-7-(3-brompropyl)-xanthins werden zusammen mit 6.9 g (36 mmol) 5-Amino-5-desoxy-1.4 ; 3.6-dianhydro-L-idit-2-nitrat und 50 ml Aethanol 24 Stdn. unter Rückfluß gekocht. Nach dem Abkühlen filtriert man von überschüssigem 5-Amino-5-desoxy-1.4 ; 3.6-dianhydro-L-idit-2-nitrat als Hydrobromid (3.5 g ; 13 mmol) ab, dampft das Filtrat unter vermindertem Druck ein, löst den Rückstand in 50 ml Chloroform, extrahiert mit 1-molarer Salzsäure noch vorhandene Ausgangsprodukte, wäscht mit 1-molarer Natronlauge säurefrei und dampft die Chloroformlösung nach dem Trocknen über wasserfreiem Natriumsulfat und Filtrieren unter vermindertem Druck ein. Man erhält 3.0 g (6.6 mmol) 5-[3-(3-Isobutyl-1-methyl-xanthin-7-yl)-propylamino]-5-desoxy-1.4 ; 3.6-dianhydro-L-idit-2-nitrat. Dieses wird in Aethanol gelöst, durch Zugabe von 6.6 mmol Salzsäure in das Hydrochlorid übergeführt, mit 1 g Aktivkohle aufgekocht, filtriert, unter vermindertem Druck eingedampft und im Vakuum (1 mbar) getrocknet. Das so erhaltene Hydrochlorid ist amorph und schmilzt unter Zersetzung bei 165-170°. $[\alpha]_D^{25}$ 29.0 (c 2 ; Methanol) und $[\alpha]_D^{25}$ 23.4 (c 0.29 ; Dimethylformamid).

Elementaranalyse : $C_{19}H_{28}N_6O_7 \times HCl$ (488.90)

| | | | | |
|---|---|---|---|---|
| Ber. : | C (46.68), | H (5.98), | N (17.19), | Cl (7.25) |
| Gef. : | C (46.51), | H (6.05), | N (16.31), | Cl (8.1 ) |

## Beispiel 9

5-[N-Methyl-N-(3-theophyllin-7-ylpropyl)-amino]-5-desoxy-1.4 ; 3.6-dianhydro-L-idit-2-nitrat :

a) 5-Methylamino-5-desoxy-1.4 ; 3.6-dianhydro-L-idit :

Die Mischung aus 22.4 g (0.1 Mol) 1.4 ; 3.6-Dianhydro-D-glucit-5-methansulfonat (Herstellung siehe Beispiel 1a) 31 g (1 Mol) Methylamin und 150 ml n-Butanol wird im geschlossenen Stahlautoklaven 15 Stdn. bei 150° unter Stickstoffatmosphäre gerührt. Nach dem Abkühlen setzt man die Lösung von 4 g (0.1 Mol) Natriumhydroxid in 200 ml n-Butanol zu, rührt durch, fällt mit 600 ml Chloroform das gebildete Natriummethansulfonat aus, filtriert und dampft das Filtrat unter vermindertem Druck ein. Die so erhaltene ölige Rohbase wird in 100 ml Isopropanol gelöst und mit 6.5 ml 65 %iger Salpetersäure in das Hydrogennitrat übergeführt. Nach dem Eindampfen unter reduziertem Druck kristallisiert man aus Isopropanol um und erhält 15.3 g (68.9 mmol) 5-Methylamino-5-desoxy-1.4 ; 3.6-dianhydro-L-idit-Hydrogennitrat.

Schmp. 108-9°. $[\alpha]_D^{25}$ 41.8 (c 1.0 ; Wasser)

Elementaranalyse : $C_7H_{13}NO_3 \times HNO_3$ (222.20)

| | | | |
|---|---|---|---|
| Ber. : | C (37.84), | H (6.35), | N (12.61) |
| Gef. : | C (38.00), | H (6.60), | N (12.23) |

b) 5-Methylamino-5-desoxy-1.4 ; 3.6-dianhydro-L-idit-2-nitrat :

Unter Rühren und Kühlen auf 10° tropft man der Mischung aus 90 ml Essigsäure, 2.3 g (38 mmol) Harnstoff und 10.2 g (46 mmol) 5-Methylamino-5-desoxy-1.4 ; 3.6-dianhydro-L-idit-Hydrogennitrat die Lösung von 5.9 ml (138 mmol) 95 %iger Salpetersäure in 46 ml Essigsäure und anschließend noch 46 ml Acetanhydrid zu. Nach weiteren 4 Stdn. Rühren bei 10° fällt man mit Aether/Petroläther das kristalline Reaktionsprodukt aus und saugt ab. Der in 200 ml Wasser gelöste Niederschlag wird durch Zugabe von Natriumhydrogencarbonat neutralisiert und die Lösung 4 mal mit je 150 ml Chloroform extrahiert. Die Chloroformextrakte werden nach dem Waschen mit 100 ml Wasser, Trocknen über wasserfreiem Natriumsulfat/Natriumcarbonat und Filtrieren unter vermindertem Druck eingedampft. Die so erhaltene Rohbase wird in 100 ml Aethanol gelöst, mit 46 ml 1-molarer Salzsäure in das Hydrochlorid übergeführt, erneut unter reduziertem Druck eingedampft und zweimal aus Aethanol/Isopropanol umkristallisiert. Man erhält 5.58 g (23.2 mmol) 5-Methylamino-5-desoxy-1.4 ; 3.6-dianhydro-L-idit-2-nitrat-Hydrochlorid.

Schmp. 163-6° (Z) ; $[\alpha]_D^{25}$ 49 (c 1.0 ; Wasser)

Elementaranalyse : $C_7H_{12}N_2O_5 \times HCl$ (240.64)

| | | | | |
|---|---|---|---|---|
| Ber. : | C (34.94), | H (5.44), | N (11.64), | Cl (14.73) |
| Gef. : | C (35.00), | H (5.57), | N (11.92), | Cl (14.8 ) |

c) 5-[N-Methyl-N-(3-theophyllin-7-ylpropyl)-amino]-5-desoxy-1.4 ; 3.6-dianhydro-L-idit-2-nitrat :

Der Mischung aus 100 ml wasserfreiem Dimethylformamid, 8.4 g (61 mmol) wasserfreiem fein gepulverten Kaliumcarbonat und 5.9 g (29 mmol) 5-Methylamino-5-desoxy-1.4 ; 3.6-dianhydro-L-idit-2-nitrat tropft man unter Rühren, Feuchtigkeitsausschluß und Erwärmen auf 50° die Lösung von 10 g (33 mmol) 7-(3-Brompropyl)-theophyllin in 60 ml wasserfreiem Dimethylformamid zu und rührt noch 4 Tage bei 50°. Man filtriert, wäscht den Filterrückstand mit 150 ml Dimethylformamid, verdünnt das Filtrat mit 300 ml Wasser, extrahiert zweimal mit je 200 ml Chloroform, wäscht die Chloroformextrakte mit 200 ml Wasser, trocknet sie über wasserfreiem Natriumsulfat, filtriert und leitet Chlorwasserstoff bis zur Sättigung ein. Mit Aether fällt man das Rohprodukt aus, das nach Umfällen aus Chloroform/Methanol mit Aether und nach Umkristallisation aus Isopropanol 11.31 g (24.5 mmol) 5-[N-Methyl-N-(3-theophyllin-7-ylpropyl)-amino]-5-desoxy-1.4 ; 3.6-dianhydro-L-idit-2-nitrat-Hydrochlorid ergibt.

Schmp. 167-9° ; $[\alpha]_D^{25}$ 22.8 (c 1.0 ; Aethanol)

Elementaranalyse : $C_{17}H_{24}N_6O_7 \times HCl$ (460.88)

| | | | | |
|---|---|---|---|---|
| Ber. : | C (44.30), | H (5.47), | N (18.29), | Cl (7.69) |
| Gef. : | C (44.42), | H (5.66), | N (17.18), | Cl (7.6 ) |

Beispiel 10

5-(3-Theophyllin-7-ylpropyl)-amino-5-desoxy-1.4 ; 3.6-dianhydro-D-glucit-2-nitrat :

a) 5-Amino-5-desoxy-1.4 ; 3.6-dianhydro-D-glucit :
Dieses Ausgangsprodukt kann auf zwei Wegen erhalten werden.

Verfahren I

Herstellung und Ammonolyse von 1.4 ; 3.6-Dianhydro-L-idit-2-methansulfonat.

Die Lösung von 73 g (480 mmol) 96 %igem 1.4 ; 3.6-Dianhydro-L-idit in 500 ml wasserfreiem Pyridin wird unter Feuchtigkeitsausschluß, Rühren und Kühlen auf – 20° tropfenweise mit 52 ml (660 mmol) 98 %igem Methansulfonylchlorid versetzt und 15 Stdn. bei – 20° nachgerührt. Unter vermindertem Druck destilliert man das Pyridin soweit wie möglich ab und erwärmt den Rückstand nach Zugabe von 500 ml heißem Wasser bis zur Lösung. Beim Abkühlen kristallisieren 47.6 g (157 mmol) 1.4 ; 3.6-Dianhydro-L-idit-2.5-dimethansulfonat aus, die abgesauft und zweimal mit je 100 ml Wasser nachgewaschen werden. Die vereinigten Filtrate werden durch Zugabe von Natriumhydrogencarbonat neutralisiert (pH = 7), unter vermindertem Druck eingedampft und getrocknet. Der gepulverte Trockenrückstand wird zweimal mit je 400 ml Chloroform ausgekocht und noch heiß filtriert. Nach dem Abkühlen des Filtrats kristallisiert 1.4 ; 3.6-Dianhydro-L-idit-2-methansulfonat aus. Die Mutterlauge liefert nach Konzentration weiteres Monomethansulfonat. Insgesamt erhält man 51.5 g (213 mmol) 1.4 ; 3.6-Dianhydro-L-idit-2-methansulfonat. Zur Analyse wird eine kleine Menge aus Methanol umkristallisiert.
Schmp. 124-5° ; $[\alpha]_D^{25}$ 33.7 (c 1.0 ; Aceton).
Elementaranalyse : $C_7H_{12}O_6S$ (224.24)

Ber. : C (37.50), H (5.40), S (14.30)
Gef. : C (37.58), H (5.53), S (14.0 )

33.6 g (150 mmol) des so erhaltenen 1.4 ; 3.6-Dianhydro-L-idit-2-methansulfonats werden zusammen mit einer Lösung von 17 g (1 Mol) Ammoniak in 250 ml n-Butanol im geschlossenen Stahlautoklaven 3 Tage auf 170° erhitzt. Nach dem Abkühlen saugt man auskristallisiertes Ammoniummethansulfonat ab und wäscht mit 100 ml n-Butanol nach. Das Filtrat wird zweimal mit je 200 ml Wasser extrahiert. Die vereinigten wäßrigen Extrakte werden mit 200 ml Chloroform gewaschen, zur Trockne eingedampft und aceotrop mit Butanol nachgetrocknet. Der Trockenrückstand wird unter Zusatz von 10 g wasserfreiem Natriumsulfat mit 50 ml n-Butanol aufgekocht, heiß filtriert und das Filtrat eingedampft. Das so erhaltene ölige Rohprodukt wird in 50 ml Chloroform aufgenommen, filtriert und eingedampft. Man erhält 14 g (96 mmol) langsam erstarrenden 5-Amino-5-desoxy-1.4 ; 3.6-dianhydro-D-glucit. Zur Charakterisierung überführt man einen kleinen Anteil des Produkts in das Hydrochlorid und kristallisiert aus Isopropanol um. Zersetzungspunkt : 240° ; $[\alpha]_D^{25}$ 39.1 (c 1.0 ; Wasser)
Elementaranalyse : $C_6H_{11}NO_3 \times HCl$ (181.63)

Ber. : C (39.68), H (6.66), N (7.71), Cl (19.52)
Gef. : C (39.85), H (6.89), N (7.66), Cl (19.3 )

Verfahren II

Herstellung und selektive Ammonolyse von 1.4 ; 3.6-Dianhydro-L-idit-2-methansulfonat-5-benzoat.

Die Mischung aus 604 g (2 Mol) 1.4 ; 3.6-Dianhydro-D-glucit-2.5-dimethansulfonat, 317 g (2.2 Mol) Natriumbenzoat und 8 Litern wasserfreiem Dimethylformamid wird 2 Tage bei 145° im Stahlautoklaven unter Stickstoffschutzatmosphäre gerührt. Unter vermindertem Druck destilliert man das Dimethylformamid ab, nimmt den Rückstand in 5 Litern Chloroform auf, extrahiert nacheinander mit je 2 Litern 1-molarer Natronlauge und Wasser, trocknet die Chloroformphase über wasserfreiem Natriumsulfat, filtriert und konzentriert auf 1 500 ml Volumen. Das beim Stehenlassen kristallisierende Rohprodukt wird abgesaugt, unter Erwärmen in 500 ml Aceton gelöst und die heiße Lösung in 1 000 ml Aethanol gegossen. Beim Abkühlen kristallisieren 273 g (0.83 Mol) 1.4 ; 3.6-Dianhydro-L-idit-2-methansulfonat-5-benzoat aus. Die Mutterlauge ergibt nach Eindampfen und Umkristallisation weitere 120 g (0.37 Mol) schwach durch Ausgangssubstanz verunreinigtes Produkt. Die analytische Probe hat nach Umkristallisation aus Aethanol den Schmp. 117° und $[\alpha]_D^{25}$ 76.6 (c 2 ; Chloroform).
Elementaranalyse : $C_{14}H_{16}O_7S$ (328.35)

Ber. : C (51.21), H (4.91), S (9.76)
Gef. : C (51.60), H (5.05), S (9.6 )

328 g (1 Mol) des so erhaltenen 1.4 ; 3.6-Dianhydro-L-idit-2-methansulfonat-5-benzoats werden zusammen mit 1 Liter Aethanol und 1.5 Liter 25 %igem wäßrigen Ammoniak 1 Tag bei 130° im geschlossenen Stahlautoklaven gerührt. Man dampft unter vermindertem Druck ein, löst den Rückstand in 1 Liter Wasser, stellt durch Zugabe von konz. Salzsäure auf pH = 1 ein und saugt den Niederschlag — bestehend aus Benzoesäure und Benzamid — ab. Das Filtrat wird nach zweimaligem Waschen mit je 500 ml Chloroform durch Zugabe von Natriumhydrogencarbonat auf pH = 8 gebracht, erneut eingedampft und der Rückstand mit 2 Litern Aethanol extrahiert. Der Aethanol-Extrakt wird nach dem Eindampfen mit 2 Litern Chloroform extrahiert, der Chloroformextrakt mit 60 g Aktivkohle aufgekocht, filtriert und eingedampft. Die so erhaltenen 105 g Rohprodukt liefern nach fraktionierter Destillation bei 0.2 Torr und 136-142° Uebergangstemperatur 86.3 g (0.59 Mol) 5-Amino-5-desoxy-1.4 ; 3.6-dianhydro-D-glucit.

b) 5-Amino-5-desoxy-1.4 ; 3.6-dianhydro-D-glucit-2-Nitrat :

55 g (0.38 Mol) des nach Verfahren I oder II erhaltenen 5-Amino-5-desoxy-1.4 ; 3.6-dianhydro-D-glucits werden durch Vermischen mit 3.5 ml Chloroform verflüssigt und diese Lösung tropfenweise unter Rühren einer auf −15° gekühlten Lösung von 14 g (0.23 Mol) Harnstoff in 202 ml (4.6 Mol) 96 %iger Salpetersäure (d = 1.5) zugefügt. Man rührt noch 15 Stdn. bei − 15° nach, verdünnt mit 750 ml Wasser und neutralisiert unter Kühlen mit einer Lösung von 168 g (4.2 Mol) Natriumhydroxid in 1.5 Litern Wasser. Mit Natriumhydrogencarbonat stellt man pH = 8 ein, filtriert und extrahiert die wäßrige Lösung 16 Stdn. lang im 5 l-Rotationsperforator (Normag) kontinuierlich mit Chloroform. Der Chloroformextrakt wird nach dem Trocknen über wasserfreiem Natriumsulfat und Filtrieren unter vermindertem Druck eingedampft. Der Rückstand wird in 200 ml Dichlormethan gelöst, durch Filtration von anorganischen Verunreinigungen befreit und erneut unter reduziertem Druck eingedampft. Man erhält 53 g (0.28 Mol) 5-Amino-5-desoxy-1.4 ; 3.6-dianhydro-D-glucit-2-nitrat als schwach gelbliches Oel. Zur Charakterisierung überführt man einen kleinen Teil in das Hydrochlorid und kristallisiert aus Aethanol/Chloroform/Petroläther um.

Schmp. 170-1° (Z) ; $[\alpha]_D^{25}$ 50.7 (C 0.53 ; Wasser)

Elementaranalyse : $C_6H_{10}N_2O_5 \times HCl$ (226.62)

| | | | | |
|---|---|---|---|---|
| Ber. : | C (31.80), | H (4.89), | N (12.36), | Cl (15.64) |
| Gef. : | C (32.00), | H (5.10), | N (12.14), | Cl (15.6 ) |

c) 5-(3-Theophyllin-7-ylpropyl)-amino-5-desoxy-1.4 ; 3.6-dianhydro-D-glucit-2-nitrat :

Zur siedenden Lösung von 19 g (100 mmol) 5-Amino-5-desoxy-1.4 ; 3.6-dianhydro-D-glucit-2-nitrat in 150 ml Aethanol gibt man über einen Soxhlet-Aufsatz 12 g (40 mmol) 7-(3-Brompropyl)-theophyllin innerhalb 6 Stdn. zu und erhitzt weitere 14 Stdn. unter Rückfluß. Man dampft unter vermindertem Druck ein und extrahiert die Lösung des Rückstands in 80 ml Chloroform nacheinander zweimal mit je 40 ml Wasser und zweimal mit je 40 ml 0.5 molarer Salzsäure. Die salzsauren Extrakte werden mit 1-molarer Natronlauge auf pH = 9 gebracht und mit Chloroform reextrahiert. Die Chloroformextrakte ergeben nach dem Trocknen über wasserfreiem Natriumsulfat, Filtrieren und Eindampfen unter reduziertem Druck 10.7 g (26 mmol) Rohbase, die in Isopropanol gelöst und durch Zugabe von 27 ml 1-molarer Salzsäure in das Hydrochlorid übergeführt wird. Erneutes Eindampfen unter reduziertem Druck und zweifache Umkristallisation aus Isopropanol ergibt 6.11 g (12.8 mmol) 5-(3-Theophyllin-7-ylpropyl)-amino-5-desoxy-1.4 ; 3.6-dianhydro-D-glucit-2-nitrat-Hydrochlorid mit 1/2 Mol Kristallisopropanol (nach Trocknen bei 70°, 1 Torr).

Schmp. 110° (nach Sintern ab 86°) ; $[\alpha]_D^{25}$ 31.9 (c 0.5 ; Wasser).

Elementaranalyse : $C_{16}H_{22}N_6O_7 \times HCl \times 1/2\ C_3H_8O$ (476.91)

| | | | | |
|---|---|---|---|---|
| Ber. : | C (44.08), | H (5.71), | N (17.62), | Cl (7.43) |
| Gef. : | C (43.91), | H (5.69), | N (16.95), | Cl (7.8 ) |

## Beispiel 11

2-(3-Theophyllin-7-ylpropyl)-amino-2-desoxy-1.4 ; 3.6-dianhydro-D-glucit-5-nitrat :

a) 1.4 ; 3.6-Dianhydro-D-mannit-2-methansulfonat :

Der Lösung von 877 g (6 Mol) 1.4 ; 3.6-Dianhydro-D-mannit in 6 Litern Pyridin tropft man unter Rühren und Feuchtigkeitsausschluß sowie kühlen auf − 15° innerhalb 6 Stdn. 525 ml (6.6 Mol) Methansulfonylchlorid zu, rührt weitere 3 Tage bei − 15° und destilliert dann unter vermindertem Druck das Pyridin ab. Beim Versetzen des öligen Rückstands mit 2.7 Litern Wasser kristallisiert reines 1.4 ; 3.6-Dianhydro-D-mannit-2.5-dimethansulfonat aus, das abgetrennt und 2 mal mit je 700 ml Wasser gewaschen wird. Die vereinigten Filtrate werden mit einer Lösung von 264 g (6.6 Mol) Natriumhydroxid in 2.5 Liter Wasser versetzt, durch Zugabe von Natriumhydrogencarbonat auf pH = 7 eingestellt, unter reduziertem Druck eingedampft und aceotrop mit Chloroform getrocknet. Der Rückstand wird zweimal mit je 2.5 Litern Chloroform heiß extrahiert und filtriert. Die vereinigten Chloroformextrakte werden 5 mal mit je 1 Liter Wasser extrahiert. Beim Konzentrieren der Wasserphasen kristallisiert das 1.4 ; 3.6-Dianhydro-D-mannit-2-methansulfonat aus. Die nach dem Absaugen verbleibende Mutterlauge ergibt nach dem Eindampfen und Umkristallisieren aus Aethanol weiteres Produkt. Restliches Produkt wird

durch Eindampfen der aethanolischen Mutterlauge, Lösen des Rückstandes in Wasser und kontinuierliche Extraktion der wäßrigen Lösung mit Chloroform im Rotationsperforator gewonnen. In der Wasserphase verbleibt nicht umgesetzter 1.4 ; 3.6-Dianhydro-D-mannit. Insgesamt erhält man 396 g (1.77 Mol) 1.4 ; 3.6-Dianhydro-D-mannit-2-methansulfonat (neben 465 g = 1.54 Mol des Dimethansulfonats). Die analytische Probe hat nach Umkristallisation aus Chloroform den Schmp. 111-112° und $[\alpha]_D^{25}$ 118 (c 1.0 ; Aceton).

Elementaranalyse : $C_7H_{12}O_6S$ (224.24)

Ber. : C (37.50), H (5.40), S (14.30)
Gef. : C (37.41), H (5.59), S (13.7 )

b) 2-Amino-2-desoxy-1.4 ; 3.6-dianhydro-D-glucit :

Die Mischung aus 224 g (1 Mol) des zuvor erhaltenen 1.4 ; 3.6-Dianhydro-D-mannit-2-methansulfonats und 1 Liter 25 %igem wäßrigen Ammoniak wird 24 Stdn. bei 120° im geschlossenen Stahlautoklaven gerührt. Nach dem Abkühlen gibt man 84 g (1 Mol) Natriumhydrogencarbonat zu, dampft unter reduziertem Druck ein und kocht den Rückstand mit 2 Litern n-Butanol aus. Der eingedampfte Butanol-Extrakt wird in 1 Liter Chloroform aufgenommen, restliches Natriummethansulfonat abfiltriert und das Filtrat eingedampft. Man erhält 130 g (0.9 Mol) 2-Amino-2-desoxy-1.4 ; 3.6-dianhydro-D-glucit als schwach gelbliches Oel. Zur Charakterisierung überführt man einen kleinen Teil in das Hydrochlorid und kristallisiert aus Isopropanol/Methanol/Chloroform um.

Schmp. 230° (Z) ; $[\alpha]_D^{25}$ 52.1 (c 1.0 ; Wasser)
Elementaranalyse : $C_6H_{11}NO_3 \times HCl$ (181.62)

Ber. : C (39.68), H (6.66), N (7.71), Cl (19.52)
Gef. : C (39.59), H (6.89), N (7.52), Cl (19.3 )

c) 2-Amino-2-desoxy-1.4 ; 3.6-dianhydro-D-glucit-5-nitrat :

Unter Rühren und Kühlen auf −15° tropft man zu 200 ml (4.5 Mol) 96 %iger Salpetersäure (d = 1.5) zunächst die Lösung von 14 g (0.23 Mol) Harnstoff in 300 ml (5.4 Mol) Schwefelsäure (d = 1.84) und anschließend innerhalb 4 Stdn. die Lösung von 145 g (1 Mol) 2-Amino-2-desoxy-1.4 ; 3-6-dianhydro-D-glucit in 50 ml Wasser und rührt 2 Stdn. bei − 15° nach. Man rührt dann in 1.5 Liter Wasser ein, stellt durch allmähliche Zugabe von 570 g (14.3 Mol) Natriumhydroxid — gelöst in 2 Litern Wasser — unter Kühlen auf pH = 8 bis 9 ein, filtriert auskristallisiertes Natriumsulfat ab, wäscht mit 500 ml Chloroform lipophile Nebenprodukte heraus und extrahiert 16 Stdn. lang im Rotationsperforator kontinuierlich mit Chloroform. Der Chloroformextrakt ergibt nach dem Trocknen über wasserfreiem Natriumsulfat, Filtrieren und Eindampfen unter vermindertem Druck 152 g (0.8 Mol) 2-Amino-2-desoxy-1.4 ; 3.6-dianhydro-D-glucit-5-nitrat als langsam erstarrendes Oel. Zur Charakterisierung wird ein kleiner Teil in das Hydrochlorid übergeführt und aus Isopropanol/Aethanol umkristallisiert.

Schmp. 181-2° (Z) ; $[\alpha]_D^{25}$ 130 (c 0.52 ; Wasser)
Elementaranalyse : $C_6H_{10}N_2O_5 \times HCl$ (226.62)

Ber. : C (31.80), H (4.89), N (12.36), Cl (15.64)
Gef. : C (31.95), H (4.90), N (12.18), Cl (15.9 )

d) 2-(3-Theophyllin-7-ylpropyl)-amino-2-desoxy-1.4 ; 3.6-dianhydro-D-glucit-5-nitrat :

Das Gemische aus 12 g (40 mmol) 7-(3-Brompropyl)-theophyllin, 19 g (100 mmol) des zuvor erhaltenen 2-Amino-2-desoxy-1.4 ; 3.6-dianhydro-D-glucit-5-nitrats und 100 ml Aethanol wird 24 Stdn. unter Rückfluß gekocht und anschließend unter reduziertem Druck eingedampft. Man nimmt in 80 ml Chloroform auf, wäscht 2 mal mit je 40 ml Wasser und extrahiert 3 mal mit je 40 ml 0.5-molarer Salzsäure. Die salzsauren Extrakte werden mit verdünnter Natronlauge auf pH = 8 bis 9 gestellt, mehrfach mit Chloroform extrahiert und die Chloroformextrakte nach dem Trocknen über wasserfreiem Natriumsulfat und Filtrieren unter vermindertem Druck eingedampft. Man erhält 22 g Rohprodukt, das in 200 ml Aethanol gelöst und durch Zugabe von 50 ml 1-molarer Salzsäure in das Hydrochlorid übergeführt wird. Man dampft erneut unter reduziertem Druck ein, kristallisiert zweimal aus Methanol um und erhält 12.5 g (28 mmol) 2-(3-Theophyllin-7-ylpropyl)-amino-2-desoxy-1.4 ; 3.6-dianhydro-D-glucit-5-nitrat-Hydrochlorid.

Schmp. 196-201° (Z) ; $[\alpha]_D^{25}$ 72.6 (c 0.41 ; Wasser)
Elementaranalyse : $C_{16}H_{22}N_6O_7 \times HCl$ (446.86)

Ber. : C (43.01), H (5.19), N (18.81), Cl (7.93)
Gef. : C (42.70), H (5.13), N (18.75), Cl (8.4 )

## Beispiel 12

5-(3-Theophyllin-7-ylpropyl)-amino-5-desoxy-1.4 ; 3.6-dianhydro-D-mannit-2-nitrat :

a) 2-Amino-2-desoxy-1.4 ; 3.6-dianhydro-D-mannit :

Das Gemisch aus 448 g (2 Mol) 1.4 ; 3.6-Dianhydro-D-glucit-2-methansulfonat (Herstellung siehe Beispiel 1a) und 1 500 ml 25 %igem wäßrigen Ammoniak wird 1 Tag bei 130° im geschlossenen Stahlautoklaven gerührt. Nach dem Abkühlen gibt man 30 g Aktivkohle zu, filtriert und extrahiert mit 1 Liter Chloroform den als Nebenprodukt gebildeten 1.4 ; 2.5 ; 3.6-Trianhydro-D-mannit [nach Eindampfen der Chloroformphase und Umkristallisation aus Aether/Petroläther insgesamt 104 g (0.81 Mol)]. Die Wasserphase wird nach dem Eindampfen unter reduziertem Druck und aceotropem Trocknen mit Aethanol und Chloroform in der Siedehitze mit 2 Litern Isopropanol extrahiert. Beim Einengen des Isopropanolextrakts auf 0.5 Liter auskristallisierendes Ammoniummethansulfonat wird abfiltriert, das Filtrat mit verdünnter Natronlauge neutralisiert, eingedampft und mit 1 Liter n-Butanol heiß extrahiert. Der Butanolextrakt wird eingedampft und der Rückstand mit 1 Liter Chloroform extrahiert. Eindampfen des filtrierten Chloroformextraktes ergibt 60 g (0.41 Mol) ölige Rohbase, die in 100 ml Essigsäure gelöst und tropfenweise mit einer Lösung von 15 ml 96 %iger Salpetersäure (d = 1.5) in 75 ml Essigsäure versetzt wird. Das auskristallisierende Hydrogennitrat wird abgesaugt und aus Isopropanol/Aethanol umkristallisiert. Man erhält 32 g (154 mmol) 2-Amino-2-desoxy-1.4 ; 3.6-dianhydro-D-mannit-Hydrogennitrat.

Schmp. 192-3° (Z) ; $[\alpha]_D^{25}$ 63.4 (c 0.51 ; Wasser)

Elementaranalyse : $C_6H_{11}NO_3 \times HNO_3$ (208.18)

Ber. : C (34.62), H (5.81), N (13.45)
Gef. : C (34.52), H (5.97), N (13.53)

b) 5-Amino-5-desoxy-1.4 ; 3.6-dianhydro-D-mannit-2-nitrat :

Zu 60 ml (1.35 Mol) 96 %iger Salpetersäure (d = 1.5) tropft man unter Rühren und Kühlen auf − 15° die Lösung von 4.2 g (70 mmol) Harnstoff in 90 ml (1.6 Mol) Schwefelsäure (d = 1.84). Bei gleicher Temperatur tropft man langsam die Lösung von 21.8 g (150 mmol) 2-Amino-2-desoxy-1.4 ; 3.6-dianhydro-D-mannit (hergestellt aus dem zuvor erhaltenen Hydrogennitrat) in 15 ml Wasser zu, rührt 2 Stdn. bei − 15° nach, gießt das Reaktionsgemisch in 1 Liter Wasser unter Rühren ein und stellt durch langsame Zugabe von 175 g (4.38 Mol) Natriumhydroxid — gelöst in 1 l Wasser — den pH = 9 ein. Anschließend extrahiert man 8 Stdn. im Rotationsperforator kontinuierlich mit Chloroform. Der Chloroformextrakt ergibt nach dem Trocknen über wasserfreiem Natriumsulfat, Filtrieren und Eindampfen unter reduziertem Druck 18.8 g (99 mmol) 5-Amino-5-desoxy-1.4 ; 3.6-dianhydro-D-mannit-2-nitrat als langsam kristallisierendes Oel. Zur Charakterisierung überführt man einen kleinen Teil in das Hydrochlorid und kristallisiert aus Aethanol um.

Schmp. 172° (Z) ; $[\alpha]_D^{25}$ 170.9 (c 0.5 ; Wasser)

Elementaranalyse : $C_6H_{10}N_2O_5 \times HCl$ (226.62)

Ber. : C (31.80), H (4.89), N (12.36), Cl (15.64)
Gef. : C (31.76), H (4.93), N (12.67), Cl (16.0 )

c) 5-(3-Theophyllin-7-ylpropyl)-amino-5-desoxy-1.4 ; 3.6-dianhydro-D-mannit-2-nitrat :

Zur unter Rückfluß siedenden Lösung von 9.5 g (50 mmol) des zuvor erhaltenen 5-Amino-5-desoxy-1.4 ; 3.6-dianhydro-D-mannit-2-nitrats in 100 ml Aethanol gibt man über einen Soxhlet-Aufsatz 6.0 g (20 mmol) 7-(3-Brompropyl)-theophyllin extraktiv zu, kocht noch 20 Stdn. unter Rückfluß, dampft unter vermindertem Druck ein, nimmt den Rückstand in 100 ml Chloroform auf und extrahiert nacheinander 2 mal mit je 100 ml Wasser, einmal mit 30 ml 0.3-molarer Essigsäure und 4 mal mit je 50 ml 0.1-molarer Salzsäure. Die salzsauren Extrakte werden mit verdünnter Natronlauge auf pH = 9 gestellt, mehrfach mit Chloroform extrahiert und die Chloroformextrakte vereinigt, über wasserfreiem Natriumsulfat getrocknet, filtriert und unter reduziertem Druck eingedampft. Man erhält 7 g (17 mmol) Rohbase. Zur Abtrennung von Disubstitutionsprodukt fraktioniert man säulenchromatographisch über 500 g Kieselgel (Woelm 63-200) mit Chloroform/Methanol 95/5 als Eluens, dampft die Fraktionen mit Rf = 0.26 (Kieselgel ; Chloroform/Methanol 9/1) ein und erhält 2.18 g (5.3 mmol) reines 5-(3-Theophyllin-7-yl-propylamino)-5-desoxy-1.4 ; 3.6-dianhydro-D-mannit-2-nitrat, das in Isopropanol gelöst, mit 5.3 ml 1-molarer Salzsäure versetzt, erneut eingedampft und zweimal aus Chloroform mit Aether umgefällt, das Hydrochlorid ergibt.

Schmp. 138-143° (Z). $[\alpha]_D^{25}$ 118.3 (c 0.41 ; Wasser)

Elementaranalyse : $C_{16}H_{22}N_6O_7 \times HCl$ (446.85)

Ber. : C (43.01), H (5.19), N (18.81), Cl (7.93)
Gef. : C (42.97), H (5.27), N (18.71), Cl (8.2 )

## Beispiel 13

5-(2-Adenin-9-yläthyl)-amino-5-desoxy-1.4 ; 3.6-dianhydro-L-idit-2-nitrat :

Die Mischung aus 7.3 g (30 mmol) 9-(2-Bromäthyl)-adenin (hergestellt durch Umsetzung von Adenin-Natriumsalz mit überschüssigem 1.2-Dibromäthan), 70 ml Aethanol und 14.3 g (75 mmol) 5-Amino-5-

desoxy-1.4 ; 3.6-dianhydro-L-idit-2-nitrat wird 30 Stdn. unter Rückfluß gekocht. Nach dem Abkühlen filtriert man, dampft das Filtrat unter reduziertem Druck ein, nimmt den Rückstand in 50 ml Chloroform auf, wäscht mit 30 ml Wasser und extrahiert 3 mal mit je 50 ml 0.2-molarer Salzsäure. Die salzsauren Extrakte werden mit verdünnter Natronlauge auf pH = 9 gestellt und mehrfach mit Chloroform extrahiert. Die vereinigten Chloroformextrakte ergeben nach dem Trocknen über wasserfreiem Natriumsulfat, Filtrieren und Eindampfen unter reduziertem Druck 10 g (28 mmol) Rohprodukt, das in 50 ml Aethanol gelöst und durch Zugabe von 60 ml 1-molarer Salzsäure in das Dihydrochlorid übergeführt wird. Man dampft erneut ein, kristallisiert zweimal aus Isopropanol/Wasser um und erhält 5.44 g (12 mmol) 5-(2-Adenin-9-yläthyl)-amino-1.4 ; 3.6-dianhydro-L-idit-2-nitrat als Dihydrochlorid mit 2 Mol Kristallwasser.

Schmp. 207-9° (Z) ; $[\alpha]_D^{25}$ 52.4 (c 0.4 ; Wasser)
Elementaranalyse : $C_{13}H_{17}N_7O_5 \times 2\ HCl \times 2\ H_2O$ (460.28)

| | | | | |
|---|---|---|---|---|
| Ber. : | C (33.92), | H (5.04), | N (21.30), | Cl (15.41) |
| Gef. : | C (33.94), | H (4.92), | N (21.24), | Cl (16.4 ) |

## Beispiel 14

5-(3-Adenin-9-ylpropyl)-amino-5-desoxy-1.4 ; 3.6-dianhydro-L-idit-2-nitrat

wird analog Beispiel 13 durch Umsetzung von 9-(3-Brompropyl)-adenin mit dem 2.5-fachen Überschuß an 5-Amino-5-desoxy-1.4 ; 3.6-dianhydro-L-idit-2-nitrat in siedendem Aethanol hergestellt. Die so erhaltene Rohbase wird mit der äquimolaren Menge Salzsäure in das Hydrochlorid übergeführt und aus Methanol umkristallisiert.

Schmp. 182-3° (Z) ; $[\alpha]_D^{25}$ 42.7 (c 1 ; Wasser)
Elementaranalyse : $C_{14}H_{19}N_7O_5 \times HCl$ (401.82)

| | | | | |
|---|---|---|---|---|
| Ber. : | C (41.85), | H (5.02), | N (24.40), | Cl (8.82) |
| Gef. : | C (41.41), | H (4.96), | N (24.10), | Cl (8.8 ) |

## Beispiel 15

5-(4-Adenin-9-ylbutyl)-amino-5-desoxy-1.4 ; 3.6-dianhydro-L-idit-2-nitrat

wird analog Beispiel 13 durch Umsetzung von 9-(4-Brombutyl)-adenin mit überschüssigem 5-Amino-5-desoxy-1.4 ; 3.6-dianhydro-L-idit-2-nitrat in siedendem Aethanol hergestellt. Die Rohbase wird mit der zweifach molaren Menge wäßriger Salzsäure in das Dihydrochlorid übergeführt, eingeengt und aus n-Propanol umkristallisiert. Das Dihydrochlorid enthält im Kristallverband je 1 Mol $H_2O$ und 1 Mol n-Propanol (Trocknung im Vakuumexsiccator bei Raumtemperatur). Oberhalb 75° gibt die Substanz allmählich Kristallwasser und Propanol ab und zersetzt sich bei 150°.

$[\alpha]_D^{25}$ 27.8 (c 0.23 ; Wasser).
Elementaranalyse : $C_{15}H_{21}N_7O_5 \times 2\ HCl \times H_2O \times C_3H_7OH$ (530.42)

| | | | | |
|---|---|---|---|---|
| Ber. : | C (40.76), | H (6.27), | N (18.49), | Cl (13.37) |
| Gef. : | C (40.74), | H (6.17), | N (18.04), | Cl (13.6 ) |

## Beispiel 16

5-(5-Adenin-9-ylpentyl)-amino-5-desoxy-1.4 ; 3.6-dianhydro-L-idit-2-nitrat

wird analog Beispiel 13 durch Umsetzung von 9-(5-Brompentyl)-adenin mit überschüssigem 5-Amino-5-desoxy-1.4 ; 3.6-dianhydro-L-idit-2-nitrat in siedendem Aethanol hergestellt. Die Rohbase wird mit der 2-fach molaren Menge Salzsäure in das Dihydrochlorid übergeführt und zweimal aus Aethanol/Isopropanol umkristallisiert. Nach Trocknung bei 110° liegt das Halbhydrat vor.

Schmp. 202-5° (Z) ; $[\alpha]_D^{25}$ 33.8 (c 0.4 ; Wasser)
Elementaranalyse : $C_{16}H_{23}N_7O_5 \times 2\ HCl \times 1/2\ H_2O$ (475.35)

| | | | | |
|---|---|---|---|---|
| Ber. : | C (40.43), | H (5.51), | N (20.63), | Cl (14.92) |
| Gef. : | C (40.72), | H (5.62), | N (20.51), | Cl (14.7 ) |

Trocknung bei 140° ergibt das wasserfreie Dihydrochlorid.
Elementaranalyse : $C_{16}H_{23}N_7O_5 \times 2\ HCl$ (466.34)

| | | | |
|---|---|---|---|
| Ber. : | C (41.21), | H (5.40), | N (21.03) |
| Gef. : | C (41.69), | H (5.50), | N (21.08) |

Beispiel 17

5-(6-Adenin-9-ylhexyl)-amino-5-desoxy-1.4 ; 3.6-dianhydro-L-idit-2-nitrat wird analog Beispiel 13 durch Umsetzung von 9-(6-Bromhexyl)-adenin mit überschüssigem 5-Amino-5-desoxy-1.4 ; 3.6-dianhydro-L-idit-2-nitrat in siedendem Aethanol hergestellt. Die Rohbase wird mit der zweifach molaren Menge Salzsäure in das Dihydrochlorid übergeführt und 2 mal aus Aethanol/Isopropanol umkristallisiert.

Schmp. 222° (Z) ; $[\alpha]_D^{25}$ 31.1 (c 0.4 ; Wasser)

Elementaranalyse (nach Trocknung bei 140°) : $C_{17}H_{25}N_7O_5 \times 2$ HCl (480.37)

Ber. : C (42.51), H (5.67), N (20.41), Cl (14.76)
Gef. : C (42.69), H (5.76), N (20.38), Cl (14.4 )

Beispiel 18

(+)- und (−)-5-[1-Methyl-3-(theophyllin-7-yl)-propylamino]-5-desoxy-1.4 ; 3.6-dianhydro-L-idit-2-nitrat :

a) (±)-5-[1-Methyl-3-(theophyllin-7-yl)-propylamino]-5-desoxy-1.4 ; 3.6-dianhydro-L-idit (Diastereomerengemisch) :

Zur Lösung von 10 g (40 mmol) 7-(3-Oxobutyl)-theophyllin in 75 ml Aethanol gibt man die Lösung von 5.8 g (40 mmol) 5-Amino-5-desoxy-1.4 ; 3.6-dianhydro-L-idit in 40 ml Methanol, setzt die Suspension von 1.5 g 10 %igem Pd/C-Katalysator in 100 ml Aethanol zu, spült mit Stickstoff und hydriert im geschlossenen Stahlautoklaven unter 50 atm Wasserstoffdruck 20 Stdn. bei Raumtemperatur und 8 Stdn. bei 50°. Nach dem Abkühlen, Entspannen und Abfiltrieren des Katalysators dampft man das Filtrat unter red. Druck ein, stellt die Lösung des Rückstands in Wasser mit Salzsäure auf pH = 2 ein und perforiert 8 Stdn. im Rotationsperforator mit Chloroform, um Nebenprodukte zu extrahieren. Man stellt die wäßrige Phase auf pH = 4 und perforiert erneut mit Chloroform. Beide Chloroformphasen werden verworfen. Nach Einstellung auf pH = 7 perforiert man 8 Stdn. mit Chloroform. Aus dieser Chloroformphase erhält man nach dem Trocken über wasserfreiem Natriumsulfat und Eindampfen unter reduziertem Druck 8.5 g (22.4 mmol) Reaktionsprodukt, aus dem Gemisch der beiden möglichen Diastereomeren bestehend. Das Gemisch wird ohne weitere Auftrennung für die folgende Veresterung mit Salpetersäure verwendet.

Zur Analyse wird ein kleiner Teil in das Hydrochlorid übergeführt und aus Isopropanol/Pentan umkristallisiert.

Schmp. 155-166° (Z). $[\alpha]_D^{25}$ 6.7 (c 0.39 ; Wasser)

Elementaranalyse : $C_{17}H_{25}N_5O_5 \times$ HCl (415.88)

Ber. : C (49.10), H (6.30), N (16.84), Cl (8.52)
Gef. : C (49.11), H (6.48), N (16.28), Cl (8.5 )

b) (+)- und (−)-5-[1-Methyl-3-(theophyllin-7-yl)-propylamino]-5-desoxy-1.4 ; 3.6-dianhydro-L-idit-2-nitrat :

Der Lösung von 3 g (50 mmol) Harnstoff in 40 ml 96 %iger Salpetersäure (d = 1.5 ; ca. 0.9 Mol) tropft man unter Rühren und Kühlen auf − 20° die Lösung von 8 g (21 mmol) des zuvor erhaltenen Diasteromerengemisches in 20 ml Methansulfonsäure zu und rührt 1 Stde. bei − 20° nach. Die Mischung wird in 220 ml Eiswasser eingerührt ; dazu wird die Lösung von 56 g (1.4 Mol) Natriumhydroxid in 150 ml Wasser getropft und die Neutralisation durch Zugabe von Natriumhydrogencarbonat vervollständigt. Man extrahiert 5 mal mit je 100 ml Chloroform, wäscht die Chloroformphasen mit 100 ml Wasser, trocknet über wasserfr. Natriumsulfat, dampft unter reduziertem Druck ein und erhält 7 g (16.5 mmol) Rohbase (Diasteromerengemisch). Diese werden säulenchromatographisch über 200 g Kieselgel mit Chloroform/Methanol 95/5 als Eluens aufgetrennt. Man erhält 3 Fraktionen :

F 1 : 2.24 g (5.28 mmol) freie Base mit Rf = 0,56 (Kieselgel ; Chloroform/Methanol 9/1). Ueberführung in das Hydrochlorid und Umkristallisation aus Aethanol liefern 1.3 g (2.8 mmol) des linksdrehenden Diasteromeren (−)-5-[1-Methyl-3-(theophyllin-7-yl)-propylamino]-5-desoxy-1.4 ; 3.6-dianhydro-L-idit-2-nitrat-Hydrochlorid vom Schmp. 201-202° (Z) und $[\alpha]_D^{25}$-13 (c 0.2 ; Wasser)

Elementaranalyse : $C_{17}H_{24}N_6O_7 \times$ HCl (460.88)

Ber. : C (44.30), H (5.47), N (18.23), Cl (7.69)
Gef. : C (44.02), H (5.51), N (18.39), Cl (7.8 )

Zwischenfraktion : 3.3 g (7.7 mmol) Diastereomerengemisch

F 2 : 0.6 g (1.4 mmol) freie Base mit Rf = 0.51 (Kieselgel ; Chloroform/Methanol 9/1). Ueberführung in das Hydrochlorid und Umkristallisation aus Essigester und nachfolgend aus Isopropanol liefern das rechtsdrehende Diastereomere (+)-5-[1-Methyl-3-(theophyllin-7-yl)-propylamino]-5-desoxy-1.4 ; 3.6-dianhydro-L-idit-2-nitrat-Hydrochlorid vom Schmp. 158-161° und $[\alpha]_D^{25}$ + 34.8 (c 0.4 ; Wasser).

Elementaranalyse : $C_{17}H_{24}N_6O_7 \times$ HCl (460.88)

Ber. : C (44.30), H (5.47), N (18.23), Cl (7.69)
Gef. : C (44.26), H (5.61), N (18.21), Cl (7.8 )

Beispiel 19

5-(2-theophyllin-7-ylpropylamino)-5-desoxy-1.4 ; 3.6-dianhydro-L-idit-2-nitrat :

a) 7-(3-Methansulfonyloxypropyl)-theophyllin :

Der Suspension von 1 190 g (5 Mol) 7-(3-Hydroxypropyl)-theophyllin in 6 500 mol wasser- und äthanolfreiem Chloroform werden 1 110 ml (8 Mol) Triäthylamin zugefügt und dazu unter Rühren, Feuchtigkeitsauschluß und Kühlen auf – 15° 580 ml (7.5 Mol) Methansulfonylchlorid getropft. Man rührt 1 Std. bei – 15° nach, läßt auf 20° kommen und rührt die Mischung in 2 Liter Eiswasser ein. Die Chloroformphase wird abgetrennt, 3 mal mit 2 Litern Wasser und 1 mal mit 2 Litern wäßriger Natriumhydrogencarbonatlösung gewaschen. Die vereinigten Waschflüssigkeiten werden mit 2 Litern Chloroform reextrahiert. Die beiden Chloroformphasen ergeben nach Trocknen über wasserfreiem Natriumsulfat, Eindampfen unter reduz. Druck und Umkristallisation des Rückstands aus Aethanol 1 372 g (4.34 Mol) 7-(3-Methansulfonyloxypropyl)-theophyllin vom Schmp. 112-113°.

Elementaranalyse : $C_{11}H_{16}N_4O_5S$ (316.34)

Ber. : C (41.77), H (5.10), N (17.71), S (10.13)
Gef. : C (41.98), H (5.15), N (17.72), S (10.1 )

b) 5-(3-Theophyllin-7-ylpropylamino)-5-desoxy-1.4 ; 3.6-dianhydro-L-idit :

Der unter Rückfluß siedenden Lösung von 406 g (2.8 Mol) 5-Amino-5-desoxy-1.4 ; 3.6-dianhydro-L-idit (hergestellt nach Beispiel 1 b) in 1 800 ml Aethanol setzt man innerhalb 6 Stdn. 333 g (1.05 Mol) 7-(3-Methansulfonyloxypropyl)-theophyllin zu, kocht weitere 18 Stdn. unter Rückfluß und dampft dann unter reduziertem Druck ein. Der Rückstand wird in 1 700 ml Wasser aufgenommen, durch Zugabe von Natriumhydroxid auf pH = 9 gestellt und das Reaktionsprodukt durch kontinuierliche Extraktion mit Chloroform im Rotationsperforator extrahiert. (Aus der Wasserphase läßt sich überschüssiges Ausgangsamin zurückgewinnen). Zur weiteren Reinigung rührt man die Chloroformphase mit 600 ml 2-molarer Salzsäure aus, verwirft die Chloroformphase und entfernt aus der salzsauren Lösung nicht umgesetztes 7-(3-Methansulfonyloxypropyl)-theophyllin, sowie Disubstitutionsprodukt durch Extraktion mit Chloroform im Rotationsperforator, wobei der pH-Wert der Wasserphase durch schrittweise Zugabe von Natriumhydroxid allmählich auf pH = 5 gesteigert wird. Anschließend stellt man die Wasserphase auf pH = 8 ein, perforiert erneut mit Chloroform, trocknet den Chloroformextrakt über wfr. Natriumsulfat, filtriert und dampft unter reduziertem Druck ein. Man erhält 316 g (0.86 Mol) ölige Rohbase. Diese wird unter Zugabe von 83 g (0.86 Mol) Methansulfonsäure aus Aethanol/Isopropanol umkristallisiert und liefert 346 g (0.75 Mol) kristallines 5-(3-Theophyllin-7-ylpropylamino)-5-desoxy-1.4 ; 3.6-dianhydro-L-idit-Hydrogenmethansulfonat vom Schmp. 145-7° und $[\alpha]_D^{25}$ 24.2 (c 0.5 ; Wasser).

Elementaranalyse : $C_{16}H_{23}N_5O_5 \times CH_3SO_3H$ (461.50)

Ber. : C (44.24), H (5.90), N (15.17), S (6.95)
Gef. : C (44.11), H (5.92), N (15.03), S (7.2 )

c) 5-(3-Theophyllin-7-ylpropylamino)-5-desoxy-1.4 ; 3.6-dianhydro-L-idit-2-nitrat :

Unter Rühren und Kühlen auf – 15° dosiert man zu 600 ml 96 %iger Salpetersäure (d = 1.5 ; ca. 13.7 Mol) 40 g (0.67 Mol) Harnstoff langsam zu, gibt anschließend 254 g (0.55 Mol) 5-(3-Theophyllin-7-ylpropylamino)-5-desoxy-1.4 ; 3.6-dianhydro-L-idit-Hydrogenmesylat in kleinen Portionen zu und rührt 1 Std. bei – 15° nach. Die Reaktionslösung wird langsam in ca. 1 400 ml Wasser eingerührt und durch Zutropfen einer Lösung von 550 g (13.75 Mol) Natriumhydroxid in 2 Litern Wasser auf pH = 8 gebracht, wobei nach Zugabe von etwa 3/4 der Natronlauge bereits 1 Liter Chloroform zugesetzt wird. Man rührt gut durch, trennt die Chloroformphase ab, extrahiert die wäßrige Phase erneut mit 1 Liter Chloroform, wäscht die vereinigten Chloroformphasen mit 1 Liter Wasser, trocknet über wasserfr. Natriumsulfat, filtriert und dampft unter reduz. Druck bei ca. 40° Badtemperatur zur Trockne ein. Die so gewonnenen 210 g (0.51 Mol) Rohbase werden in 700 ml Aethanol gelöst und mit einer Mischung aus 45 ml 37 %iger Salzsäure (0.54 Mol) und 200 ml Aethanol versetzt. Absaugen und Waschen des kristallinen Niederschlags mit 3 mal 100 ml Aethanol und 1 mal 100 ml Chloroform ergeben nach Trocknen im Vakuumschrank bei 50° insgesamt 200 g (0.448 Mol) 5-3-Theophyllin-7-ylpropylamino)-5-desoxy-1.4 ; 3.6 dianhydro-L-idit-2-nitrat-Hydrochlorid vom Schmp. 212-14° (Z) und $[\alpha]_D^{25}$ 29.2 (c 0.5 ; Wasser).

Elementaranalyse : $C_{16}H_{22}N_6O_7 \times HCl$ (446.85)

Ber. : C (43.01), H (5.19), N (18.81), Cl (7.93)
Gef. : C (43.10), H (5.33), N (18.84), Cl (8.1 )

Als Vergleichsverbindungen wurden bei der Untersuchung der pharmakologischen Eigenschaften

der erfindungsgemäßen Verbindungen stets die im Handel erhältlichen Verbindungen Isosorbiddinitrat (ISDN) und Isosorbidmononitrat (ISMN) verwendet, wobei es sich bei ISMN um das 1.4 ; 3.6-Dianhydro-D-glucit-2-nitrat handelt.

Die koronardurchflußsteigernde Wirksamkeit der erfindungsgemäßen wurde an isolierten Meerschweinchenherzen (isolierte Herzen nach Langendorff, Methode nach Bunger et al. ; Pflüger's Archiv, *353,* 317-325 (1975)) ermittelt. Nach Erreichen des stationären Zustands in der 30. Minute wurden die Herzen mit je 50 ml Tyrodelösung mit einem Gehalt an Prüfsubstanz von jeweils 25 µg/ml perfundiert. Jede Prüfsubstanz wurde an 3-6 Herzen geprüft.

Gemessen wurde jeweils die Inotropie, der Durchfluß und die Frequenz, wobei die in der Tabelle 1 angegebenen Werte Mittelwerte der prozentualen Aenderungen gegenüber dem Vorlauf sind. Der Vergleich der gemessenen Werte zeigt, daß die koronardurchflußsteigernde Wirksamkeit der erfindungsgemäßen Verbindungen größer als diejenige von ISMN ist.

Tabelle I

Versuche am Langendorff — Herzen

Die in der Tabelle aufgeführten Werte zeigen die prozentuale Änderung gegenüber dem Vorlauf

| Substanz gemäß Beispiel Nr. | | Inotropie | Durchfluß | Frequenz |
|---|---|---|---|---|
| ISMN | | - 2,67 | + 9,11 | - 0,51 |
| 1d | | - 1,29 | + 56,42 | - 1,95 |
| 2 | | - 4,60 | + 34,69 | + 3,50 |
| 3 | | - 3,82 | + 36,41 | - 4,80 |
| 4 | | - 5,44 | + 55,67 | - 8,41 |
| 5 | | - 43,33 | + 61,90 | - 11,10 |
| 6 | | + 4,50 | + 53,31 | + 3,39 |
| 7c | | + 10,50 | + 28,90 | ± 0 |
| 9c | | - 4,3 | + 40,54 | ± 0 |
| 10c | | - 6,4 | + 13,00 | - 5,6 |
| 13 | | + 2,3 | + 12,20 | ± 0 |
| 14 | | + 3,8 | + 55,80 | ± 0 |
| 15 | | + 3,85 | + 47,10 | - 3,45 |
| 16 | | + 6,50 | + 22,50 | - 6,80 |
| 17 | | - 11,50 | + 60,60 | - 3,60 |

Die spasmolytische Wirksamkeit der erfindungsgemäßen Verbindungen wurde an isolierten Ratten-Aorta-Präparaten mit Noradrenalin-induzierten Kontraktionen bestimmt (Methode nach Wende und

Peiper, Flüger's Archiv *320*, 133-141 (1970) ; und Towart und Stoepel, Naunyn Schmiedeberg's Archives of Pharmacology ; suppl. Vol. *308*, R 18 (1979)).

In Tabelle II sind die Konzentrationen der Prüfsubstanzen angegeben, die für 50 % Hemmung des Spasmus notwendig sind ($ED_{50}$-Werte). Die spasmolytischen Wirksamkeiten der erfindungsgemäßen Verbindungen sind ganz überwiegend besser als diejenigen von ISMN und ISDN, besonders wenn man das pharmakologisch wichtige Verhältnis der effektiven Dosen beim Noradrenalinspasmus und Kaliumchloridspasmus berücksichtigt.

Die blutdrucksenkende Wirksamkeit der erfindungsgemäßen Verbindungen wurde im Vergleich zu ISDN und ISMN an narkotisierten Meerschweinchen nach i. v. Gabe gemessen. Die in Tabelle III wiedergegebenen Werte zeigen, daß die erfindungsgemäße Verbindung 1d wirksamer als ISMN oder ISDN ist.

Tabelle II

| $ED_{50}$-Werte in Mol/l für spasmolytische Wirkungen | | |
|---|---|---|
| Substanz gemäß Beispiel Nr. | Noradrenalinspasmus | Kaliumchloridspasmus |
| ISDN | $1,30 \times 10^{-6}$ | $3,10 \times 10^{-6}$ |
| ISMN | $1,60 \times 10^{-5}$ | $2,40 \times 10^{-6}$ |
| 1d | $5,60 \times 10^{-7}$ | $2,90 \times 10^{-6}$ |
| 2 | $4,20 \times 10^{-6}$ | $3,05 \times 10^{-5}$ |
| 3 | $2,60 \times 10^{-6}$ | $3,60 \times 10^{-6}$ |
| 4 | $2,70 \times 10^{-6}$ | $3,80 \times 10^{-6}$ |
| 5 | $3,60 \times 10^{-7}$ | $2,40 \times 10^{-6}$ |
| 6 | $1,12 \times 10^{-6}$ | $1,80 \times 10^{-5}$ |
| 9c | $2,95 \times 10^{-6}$ | $4,59 \times 10^{-5}$ |
| 14 | $1,40 \times 10^{-6}$ | $4,39 \times 10^{-5}$ |

Tabelle III

Blutdruckversuche an Meerschweinchen

| Substanz | Dosis mg/kg | Blutdruck vorher mm Hg | Blutdruck nachher mm Hg | Δ mm Hg |
|---|---|---|---|---|
| ISDN | 0,25 | 68,70 ± 2,30 | 57,00 ± 2,10 | - 11,70 |
| | 1,00 | 66,00 ± 3,50 | 46,30 ± 0,90 | - 19,70 |
| | 2,50 | 66,70 ± 1,70 | 37,70 ± 0,90 | - 29,00 |
| ISMN | 0,25 | 57,60 ± 3,10 | 53,90 ± 3,10 | - 3,70 |
| | 1,00 | 54,70 ± 3,80 | 48,40 ± 3,10 | - 6,30 |
| | 2,50 | 52,30 ± 4,80 | 41,40 ± 3,90 | - 10,90 |
| 1d | 0,25 | 67,75 ± 3,27 | 49,75 ± 2,78 | - 18,00 |
| | 1,00 | 68,00 ± 3,85 | 40,00 ± 2,12 | - 28,00 |
| | 2,50 | 66,25 ± 2,66 | 37,50 ± 1,76 | - 28,75 |

Die inotrope und frequenzsenkende Herzkreislaufwirksamkeit der erfindungsgemäßen Verbindungen wurde an mischrassigen Katzen von 2,5 bis 3,5 kg Körpergewicht bei intravenöser Applikation bestimmt. Die Tiere wurden mit einem Gemisch von Chloralose-Urethan narkotisiert (1,2 g/kg Urethan + 40 mg/kg Chloralose i. p. verabreicht). Sie atmeten spontan durch eine Tracheakanüle. Die A. Carotis sinistra wurde benutzt, um ein Katheter-Tip-Manometer in die linke Herzkammer zu legen. Die V. Jugularis diente zu Injektionswecken. Ueber die A. Femoralis dextra wurde ein Katheter bis in die Aorta descendans vorgeschoben und an einen Druckaufnehmer (Statham P 23Db) angeschlossen. Die Herzfrequenz wurde mit einem Pulsfrequenzmesser (Fa. Hugo Sachs Elektronik) aus dem linksventrikulären Drucksignal registriert.

Wie sich aus den in der Tabelle IV angegebenen Werten ergibt, ist die Wirksamkeit der geprüften erfindungsgemäßen Verbindung besser als diejenige der Vergleichsverbindung ISDN.

(Siehe Tabelle IV Seite 23 f.)

Tabelle IV

| Substanz | Dosis mg/kg | Herzfrequenz vorher | Herzfrequenz nachher | Herzfrequenz Δ | Blutdruck vorher | Blutdruck nachher | Blutdruck Δ | Blutdruck WD min | dp/dt vorher | dp/dt nachher | dp/dt Δ | dp/dt WD min |
|---|---|---|---|---|---|---|---|---|---|---|---|---|
| ISDN | 0,25 | 165,33<br>± 7,96 | 171,67<br>± 6,62 | + 6,34 | 98,83<br>± 6,07 | 81,17<br>± 7,98 | - 17,66 | 13,00<br>± 4,52 | 7650<br>± 585 | 8050<br>± 1000 | + 400<br>--- | |
| | 0,50 | 167,00<br>± 8,18 | 173,33<br>± 5,21 | + 6,33 | 95,83<br>± 4,56 | 73,67<br>± 4,98 | - 22,16 | 16,50<br>± 6,91 | 7333<br>± 344 | 7667<br>± 1003 | + 334 | |
| | 1,00 | 166,67<br>± 9,53 | 174,33<br>± 7,20 | + 7,66 | 96,50<br>± 3,88 | 69,17<br>± 3,76 | - 27,33 | 16,00<br>± 3,41 | 6983<br>± 367 | 7583<br>± 1231 | + 600 | |
| | 2,50 | 162,00<br>± 8,18 | 170,67<br>± 5,83 | + 8,67 | 92,50<br>± 4,37 | 60,67<br>± 3,55 | - 31,83 | >10 | 6450<br>± 545 | 7567<br>± 1540 | + 1117 | |
| 1d | 0,25 | 180,86<br>± 7,06 | 184,57<br>± 7,42 | + 3,71 | 103,00<br>± 5,01 | 64,86<br>± 4,51 | - 38,14 | 27,71<br>± 5,38 | 7371<br>± 997 | 6086<br>± 967 | - 1285 | 15,57<br>± 5,68 |
| | 0,50 | 178,29<br>± 6,74 | 182,29<br>± 7,92 | + 4,00 | 101,86<br>± 5,95 | 61,57<br>± 4,92 | - 40,29 | 20,21<br>± 5,39 | 6614<br>± 1006 | 5300<br>± 988 | - 1314 | 24,57<br>± 7,02 |
| | 1,00 | 174,00<br>± 8,09 | 182,00<br>± 9,49 | + 8,00 | 103,43<br>± 7,96 | 59,86<br>± 5,67 | - 43,57 | 24,29<br>± 7,60 | 5957<br>± 774 | 5186<br>± 1005 | - 771 | 19,58<br>± 9,35 |
| | 2,50 | 169,67<br>± 9,60 | 180,33<br>±11,24 | + 10,66 | 107,50<br>± 8,80 | 63,88<br>± 7,09 | - 43,62 | >10 | 6383<br>± 556 | 6550<br>± 1074 | + 167 | 6,42<br>± 1,62 |

Legende: Herzfrequenz, Blutdruck und Inotropie nach intravenöser Applikation von ISDN oder Substanz 1d an Katzen.

Mittelwerte ± Standardfehler aus je 6 tieren

Die inotrope und frequenzsenkende Herzkreislaufwirksamkeit der erfindungsgemäßen Verbindungen wurde weiterhin an mischrassigen Katzen von 2,5 bis 3,5 kg Körpergewicht bei intraduodenaler Applikation bestimmt. Die Tiere wurden mit einem Gemisch von Chloralose-Urethan narkotisiert (1.2 g/kg Urethan + 40 mg/kg Chloralose i. p. verabreicht). Sie atmeten spontan durch eine Tracheakanüle. Die A. Carotis sinistra wurde benutzt, um ein Katheter-Tip-Manometer in die linke Herzkammer zu legen. Die V. Jugularis diente zu Injektionszwecken. Ueber die A. Femoralis wurde ein Katheter bis in die Aorta descendans vorgeschoben und an einen Druckaufnehmer (Statham P 23Db) angeschlossen. Die Herzfrequenz wurde mit einem Pulsfrequenzmesser (Fa. Hugo Sachs Elektronik) aus dem linksventrikulären Drucksignal registriert. Durch eine Laparatomie wurde eine Duodenalschlinge freigelegt. Die zu prüfenden Substanzen wurden direkt ins Lumen injiziert.

Wie sich aus den in der Tabelle V angegebenen Werten ergibt, ist die Wirksamkeit der geprüften erfindungsgemäßen Verbindung besser als diejenige der Vergleichsverbindung ISDN.

(Siehe Tabelle V Seite 25 f.)

Tabelle V

| Substanz + Dosis | Zeit (min) nach Appl. | Frequenz $min^{-1}$ | Blutdruck mm Hg | Δ | dp/dt mm sec, | Δ |
|---|---|---|---|---|---|---|
| ISDN 5 mg/kg | 0 | 174,3 ± 9,3 | 104,3 ± 7,7 | | 8800 ± 831 | |
| | 10 | 179,3 ± 7,7 | 90,4 ± 12,5 | - 13,9 | 8000 ± 1097 | - 800 |
| | 30 | 177,7 ± 7,7 | 91,2 ± 10,0 | - 13,1 | 766 ± 807 | -1133 |
| | 60 | 175,0 ± 8,6 | 94,2 ± 9,2 | - 10,1 | 7633 ± 743 | -1167 |
| | 120 | 166,7 ± 9,4 | 95,0 ± 9,6 | - 9,3 | 7050 ± 661 | -1750 |
| 1 d 5 mg/kg | 0 | 173,2 ± 14,5 | 115,4 ± 14,4 | | 6000 ± 1367 | |
| | 10 | 173,6 ± 14,8 | 86,0 ± 13,8 | - 29,4 | 4880 ± 1371 | - 1120 |
| | 30 | 166,0 ± 15,6 | 88,6 ± 14,8 | - 26,8 | 4500 ± 1312 | - 1440 |
| | 60 | 168,4 ± 14,0 | 95,0 ± 14,4 | - 20,4 | 5020 ± 1330 | - 980 |
| | 120 | 164,8 ± 13,2 | 99,4 ± 13,2 | - 16,0 | 5100 ± 1341 | - 900 |

Legende : Herzfrequenz, Blutdruck und Inotropie zu verschiedenen Zeiten nach intraduodenaler Verabreichung von ISDN oder Substanz 1d an Katzen.

Mittelwerte ± Standardfehler aus je 6 Tieren

Die kreislaufpharmakologische Wirksamkeit der erfindungsgemäßen Verbindungen wurde am narkotisierten Hund bei intravenöser Applikation bestimmt. Die Untersuchungen wurden an gemischtrassigen Hunden beiderlei Geschlechtes, Körpergewicht 20,5 bis 29 kg, durchgeführt. Nach einer zwölfstündigen Nüchternperiode erhielten die Tiere am Morgen des Versuchstages 2 mg/kg Morphin s. c. Etwa 30 Minuten danach wurde die Narkose mit 25 mg/kg Nembutal i. v. eingeleitet und durch kontinuierliche Infusion mit 5 mg/kg/h fortgeführt. Die Tiere wurden durch einen Trachealtubus mit einem $N_2O/O_2$-Gemisch (2 : 1) konstant beatmet. Der $CO_2$-Gehalt der Ausatemluft wurde kontinuierlich, der pH-Wert des Blutes etwa alle 20 Minuten gemessen.

Die Arteria saphena dextra wurde zur fortlaufenden Messung des arteriellen Blutdruckes freigelegt, die Arteria femoralis sinistra zur Anlegung eines Statham-Flußmeßkopfes. Von der Arteria brachialis sinistra wurde eine Thermosonde in den Aortenbogen vorgeschoben, sie diente der Messung der Herzminutenvolumens mittels der Thermodilutionsmethode. Unter Röntgenkontrolle wurde durch die Arteria carotis dextra ein Katheter-Tip-Manometer in den linken Ventrikel eingebracht und damit der linksventrikulär-enddiastolische Druck ermittelt. Von der rechten Vena jugularis wurde ein doppellumiger Katheter in die Arteria pulmonalis eingebracht ; über ein Lumen wurde eisgekühlte isotone NaCl-Lösung zur Bestimmung des Herzminutenvolumens eingespritzt, über das andere der Blutdruck in der Arteria pulmonalis gemessen. Von einer Vene des linken Vorderfußes wurde ein Katheter in den rechten Vorhof zur Druckmessung eingeschoben. Mit dem differentiator wurde aus dem Druck im linken Ventrikel die Druckanstiegsgeschwindigkeit (« dp/dt ») als Maß für die Kontraktilität errechnet. Nach Beendigung der Präparation wurden die Tiere mit 500 I. E./kg Heparin antikoaguliert. Nach einer Stabilisationsperiode von etwa 30 Minuten wurde das Herzminutenvolumen bestimmt. Dann erhielten je vier Tiere 1 mg/kg ISDN bzw. 9c, 0.5 mg/kg 1d i. v. verabreicht. Die Parameter Herzfrequenz, arterieller Blutdruck, Pulmonalisdruck, Druck im rechten Vorhof, linksventrikulärer Druck, dp/dt und mittlerer Femoralisfluß wurden mit einem Beckman-8-Kanalschreiber fortlaufend registriert. Weiter wurde zur 2., 5., 7., 10., 15., 20., 25., 30., 40., 50., 60., 120., 150., und 180. Minute das Herzminutenvolumen gemessen. All Flußgrößen und daraus abgeleiteten Größen wurden auf ein Körpergewicht von 20 kg bezogen. Herzleistungen und Widerstände wurden in der üblichen Art errechnet.

Wie sich aus den in den Tabellen VI, VII und VIII angegebenen Werten ergibt, ist die Wirksamkeit der erfindungsgemäßen Verbindung besser als diejenige der Vergleichsverbindung ISDN. Insbesondere wird eine langanhaltende Senkung des arteriellen Druckes und des enddiastolischen Druckes im linken Ventrikel erreicht.

Legende zu den Tabellen VI, VII und VIII

Kreislaufpharmakogische Wirkungen von ISDN (Tab. VI), 1d (Tab. VII) und 9c (Tab. VIII) am Hund nach intravenöser Applikation (1 mg/kg, ISDN und 9c ; 0.5 mg/kg, 1d). Mittelwerte und Standardfehler aus je 4 Tieren vor der Applikation (v) bzw. verschiedene Zeiten nach der Applikation. HF : Herzfrequenz ($min^{-1}$) ; $AP_{syst.}$, $AP_{\underline{diast.}}$ : Systolischer und diastolischer Aortendruck (mm Hg) ; PRV : Druck im rechten Vorhof (mm Hg) ; $\overline{PAP}$ : Mittlerer Druck in der A. Pulmonalis (mm Hg) ; LVEDP : Enddiastolischer Druck im linken Ventrikel (mm Hg) ; dp/dt : Druckanstiegsgeschwindigkeit im linken Ventrikel (mm Hg/sec) ; $HL_L$ : Herzleistung links, bezogen auf 20 kg Körpergewicht (W) ; $HL_R$ : Herzleistung rechts, bezogen auf 20 kg Körpergewicht (mW) ; SV: Schlagvolumen, bezogen auf 20 kg Körpergewicht (ml) ; $BF_{Fem}$ : Blutfluß in der A. Femoralis bezogen auf 20 kg Körpergewicht (ml/min) ; $W_{Fem}$ : Femoraliswiderstand, bezogen auf 20 kg KG (KU) ; HZV : Herzminutenvolumen, bezogen auf 20 kg KG (1/min) ; $W_{AP}$ : Pulmonaliswiderstand, bezogen auf 20 kg KG (U) ; $W_{TP}$ : Gesamter peripherer Widerstand, bezogen auf 20 kg Körpergewicht (U).

(Siehe Tabelle VI Seite 27 f.)

Tabelle VI : ISDN (Vergleich)

| | v | 2 | 5 | 7 | 10 | 15 | 20 | 25 |
|---|---|---|---|---|---|---|---|---|
| HF | 109,5 ± 7,1 | 143,7 ± 5,1 | 133,5 ±11,9 | 128,7 ±11,1 | 121,7 ±10,6 | 118,2 ± 9,8 | 112,5 ± 8,8 | 112,0 ± 8,5 |
| $AP_{syst}$ | 166,9 ± 4,9 | 145,7 ± 7,9 | 152,9 ± 6,4 | 152,9 ± 6,5 | 152,9 ± 5,6 | 154,8 ± 4,7 | 154,8 ± 5,6 | 156,6 ± 4,7 |
| $AP_{diast}$ | 95,6 ±10,8 | 99,4 ± 9,7 | 102,2 ± 7,7 | 100,3 ± 8,0 | 98,4 ± 9,0 | 98,4 ± 9,0 | 96,6 ± 7,4 | 98,4 ± 7,2 |
| PRV | 2,06 ± 0,5 | 1,12 ± 0,6 | 1,72 ± 0,7 | 1,78 ± 0,7 | 1,87 ± 0,6 | 1,97 ± 0,7 | 2,15 ± 0,7 | 2,38 ± 0,8 |
| $\overline{PAP}$ | 14,3 ± 1,2 | 11,1 ± 0,7 | 11,5 ± 0,8 | 12,6 ± 1,4 | 12,6 ± 1,4 | 12,7 ± 1,6 | 12,6 ± 1,3 | 13,3 ± 1,5 |
| LVEDP | 7,69 ± 1,3 | 4,97 ± 1,1 | 5,81 ± 0,8 | 6,0 ± 0,9 | 6,28 ± 0,8 | 6,47 ± 0,7 | 6,75 ± 0,7 | 7,03 ± 0,7 |
| dp/dt | 2268,7 ±271,1 | 2868,7 ±370,4 | 2606,2 ± 30,1 | 2587,5 ±291,0 | 2550,0 ±302,2 | 2512,5 ±288,0 | 2418,7 ±281,2 | 2456,2 ±295,9 |
| $HL_L$ | 0,76 ±0,13 | 0,94 ±0,15 | 0,95 ±0,17 | 0,85 ±0,14 | 0,84 ±0,15 | 0,79 ±0,14 | 0,78 ±0,13 | 0,76 ±0,13 |
| $HL_R$ | 85,1 ±19,8 | 85,3 ±11,3 | 81,4 ±13,9 | 83,0 ±18,3 | 82,4 ±17,9 | 78,9 ±21,3 | 75,3 ±16,3 | 76,3 ±18,2 |
| SV | 27,7 ± 2,8 | 26,3 ± 1,8 | 27,4 ± 2,1 | 25,9 ± 1,5 | 27,4 ± 1,9 | 26,8 ± 2,3 | 27,9 ± 2,3 | 26,8 ± 2,1 |
| $BF_{Fem}$ | 44,9 ±11,7 | 31,2 ± 6,5 | 35,4 ± 7,7 | 37,3 ± 7,1 | 38,4 ± 6,6 | 40,9 ± 8,6 | 40,9 ± 7,2 | 41,9 ± 8,5 |
| $W_{Fem}$ | 0,39 ±0,1 | 0,55 ±0,1 | 0,49 ±0,1 | 0,45 ±0,1 | 0,42 ±0,05 | 0,41 ±0,1 | 0,40 ±0,05 | 0,40 ±0,1 |
| HZV | 3,03 ±0,4 | 3,8 ±0,4 | 3,7 ±0,5 | 3,3 ±0,4 | 3,4 ±0,4 | 3,2 ±0,4 | 3,1 ±0,4 | 3,0 ±0,4 |
| $W_{AP}$ | 0,31 ±0,05 | 0,23 ±0,05 | 0,22 ±0,04 | 0,27 ±0,03 | 0,26 ±0,03 | 0,26 ±0,03 | 0,25 ±0,02 | 0,27 ±0,02 |
| $W_{TP}$ | 5,27 ±0,4 | 4,02 ±0,1 | 4,39 ±0,4 | 4,72 ±0,3 | 4,66 ±0,3 | 4,96 ±0,3 | 4,92 ±0,3 | 5,22 ±0,4 |

Tabelle VI (Fortsetzung) : ISDN (Vergleich)

| | 30 | 40 | 50 | 60 | 90 | 120 | 150 | 180 |
|---|---|---|---|---|---|---|---|---|
| HF | 110,0 ±17,5 | 108,0 ± 9,8 | 109,2 ±21,2 | 107,5 ±10,1 | 109,5 ± 9,9 | 104,2 ± 7,7 | 103,7 ± 6,9 | 103,7 ± 5,9 |
| $AP_{syst}$ | 155,6 ± 3,2 | 158,4 ± 4,9 | 157,5 ± 5,1 | 159,4 ± 5,8 | 163,2 ± 5,8 | 161,2 ± 6,6 | 161,2 ± 4,7 | 161,2 ± 4,8 |
| $AP_{diast}$ | 98,1 ± 5,9 | 101,2 ± 6,5 | 101,2 ± 6,5 | 101,2 ± 6,5 | 99,4 ± 5,6 | 97,5 ± 5,5 | 95,6 ± 4,5 | 95,6 ± 5,6 |
| PRV | 2,34 ±0,7 | 2,34 ±0,7 | 2,29 ±0,6 | 2,53 ±0,7 | 2,53 ±0,7 | 2,81 ±0,8 | 3,0 ±0,9 | 2,81 ±0,8 |
| $\overline{PAP}$ | 13,5 ± 1,5 | 14,0 ± 1,5 | 14,0 ± 1,6 | 14,1 ± 1,6 | 14,6 ± 1,8 | 15,0 ± 2,0 | 14,6 ± 1,5 | 15,5 ± 1,6 |
| LVEDP | 7,22 ±0,6 | 7,60 ±0,7 | 7,60 ±0,7 | 7,77 ±0,7 | 7,98 ±0,9 | 8,34 ±1,0 | 7,78 ±1,0 | 7,60 ±1,2 |
| dp/dt | 2390,6 ±284,9 | 2343,7 ±261,2 | 2306,2 ±269,2 | 2250,0 ±250,6 | 2231,2 ±251,2 | 2118,7 ±221,5 | 2025,0 ±181,1 | 2043,7 ±206,2 |
| $HL_L$ | 0,76 ±0,10 | 0,76 ±0,13 | 0,77 ±0,13 | 0,78 ±0,14 | 0,77 ±0,12 | 0,72 ±0,10 | 0,74 ±0,10 | 0,79 ±0,12 |
| $HL_R$ | 78,6 ±16,5 | 80,5 ±17,2 | 81,1 ±18,6 | 81,9 ±20,1 | 85,4 ±20,5 | 81,5 ±18,9 | 79,7 ±17,2 | 92,4 ±18,7 |
| SV | 27,8 ± 2,1 | 27,7 ± 1,9 | 27,4 ± 1,6 | 28,2 ± 1,6 | 27,7 ± 1,9 | 28,1 ± 1,8 | 28,6 ± 1,6 | 30,6 ± 1,9 |
| $BF_{Fem}$ | 41,5 ± 8,1 | 42,9 ± 8,2 | 43,9 ± 9,2 | 42,3 ± 8,9 | 44,4 ± 8,2 | 49,3 ± 7,7 | 51,3 ± 9,8 | 53,7 ±10,8 |
| $W_{Fem}$ | 0,40 ±0,1 | 0,40 ±0,05 | 0,39 ±0,1 | 0,41 ±0,1 | 0,38 ±0,04 | 0,33 ±0,03 | 0,32 ±0,05 | 0,32 ±0,06 |
| HZV | 3,0 ±0,3 | 3,0 ±0,3 | 3,0 ±0,4 | 3,0 ±0,4 | 3,0 ±0,4 | 2,9 ±0,2 | 3,0 ±0,3 | 3,2 ±0,3 |
| $W_{AP}$ | 0,27 ±0,03 | 0,28 ±0,04 | 0,27 ±0,04 | 0,26 ±0,03 | 0,27 ±0,06 | 0,28 ±0,08 | 0,29 ±0,05 | 0,31 ±0,05 |
| $W_{TP}$ | 5,13 ±0,4 | 5,41 ±0,5 | 5,38 ±0,5 | 5,32 ±0,5 | 5,35 ±0,6 | 5,36 ±0,3 | 5,22 ±0,3 | 4,89 ±0,3 |

Tabelle VII : Verbindung gemäß Beispiel 1d

| | v | 2 | 5 | 7 | 10 | 15 | 20 | 25 |
|---|---|---|---|---|---|---|---|---|
| HF | 101,2 ± 9,6 | 145,7 ±24,1 | 131,2 ±20,3 | 119,5 ±16,5 | 110,2 ±14,5 | 107,7 ±13,1 | 104,0 ±10,1 | 103,7 ± 8,9 |
| $AP_{syst}$ | 152,8 ±12,0 | 107,8 ± 8,0 | 119,4 ± 5,2 | 122,8 ± 6,4 | 124,7 ± 8,3 | 126,0 ± 6,3 | 127,9 ± 6,6 | 127,5 ± 5,5 |
| $AP_{diast}$ | 76,9 ± 4,5 | 67,5 ± 3,4 | 77,8 ± 5,2 | 75,9 ± 5,2 | 74,6 ± 5.5 | 75,9 ± 3,2 | 78,7 ± 2,6 | 79,7 ± 1,8 |
| PRV | 3,09 ±0,56 | 0,37 ±0,15 | 0,60 ±0,20 | 1,12 ±0,37 | 1,22 ±0,41 | 1,59 ±0,41 | 1,78 ±0,46 | 2,25 ±0,63 |
| $\overline{PAP}$ | 15,59 ±1,19 | 11,25 ±1,03 | 11,44 ±1,22 | 11,75 ±1,40 | 11,90 ±1,43 | 12,19 ±1,38 | 12,37 ±1,08 | 12,53 ±0,85 |
| LVEDP | 7,50 ±0,61 | 3,85 ±0,23 | 4,78 ±0,32 | 5,16 ±0,46 | 4,69 ±0,24 | 4,97 ±0,28 | 5,62 ±0,34 | 5,44 ±0,32 |
| dp/dt | 1903,1 ±188,3 | 2915,6 ±247,0 | 2671,9 ±185,8 | 2662,5 ±206,5 | 2465,6 ±396,9 | 2465,6 ±389,8 | 2428,1 ±385,1 | 2353,1 ±371,0 |
| $HL_L$ | 0,50 ±0,09 | 0,51 ±0,09 | 0,47 ±0,07 | 0,47 ±0,06 | 0,47 ±0,07 | 0,44 ±0,06 | 0,45 ±0,06 | 0,45 ±0,06 |
| $HL_R$ | 67,1 ±12,7 | 70,9 ±10,6 | 58,2 ± 9,5 | 56,8 ± 9,2 | 57,1 ±10,3 | 53,8 ±10,1 | 52,8 ± 8,1 | 50,9 ± 7,1 |
| SV | 23,4 ± 2,5 | 21,9 ± 4,3 | 19,6 ± 3,5 | 21,3 ± 3,5 | 22,9 ± 3,6 | 22,0 ± 3,6 | 22,3 ± 3,4 | 22,3 ± 3,6 |
| $BF_{Fem}$ | 51,5 ±10,8 | 42,6 ±10,0 | 40,8 ± 8,2 | 42,7 ± 7,7 | 46,2 ± 8,6 | 48,3 ± 9,7 | 51,5 ±10,5 | 52,9 ± 9,0 |
| $W_{Fem}$ | 0,29 ±0,05 | 0,28 ±0,05 | 0,32 ±0,05 | 0,30 ±0,04 | 0,28 ±0,05 | 0,28 ±0,06 | 0,27 ±0,05 | 0,27 ±0,05 |
| HZV | 2,35 ±0,29 | 2,94 ±0,34 | 2,40 ±0,24 | 2,40 ±0,22 | 2,38 ±0,23 | 2,26 ±0,25 | 2,24 ±0,25 | 2,24 ±0,29 |
| $W_{AP}$ | 0,46 ±0,04 | 0,35 ±0,06 | 0,38 ±0,07 | 0,37 ±0,08 | 0,41 ±0,07 | 0,43 ±0,07 | 0,41 ±0,06 | 0,44 ±0,07 |
| $W_{TP}$ | 5,78 ±0,53 | 3,73 ±0,23 | 5,16 ±0,40 | 5,10 ±0,38 | 5,10 ±0,35 | 5,51 ±0,47 | 5,71 ±0,47 | 5,78 ±0,64 |

Tabelle VII (Fortsetzung) : Verbindung gem. Beispiel 1d

| | 30 | 40 | 50 | 60 | 90 | 120 | 150 | 180 |
|---|---|---|---|---|---|---|---|---|
| HF | 101,2 ± 8,3 | 97,5 ± 7,2 | 96,2 ± 6,9 | 95,5 ± 5,3 | 92,5 ± 7,6 | 96,5 ±10,5 | 104,5 ± 7,3 | 114,0 ± 6,0 |
| $AP_{syst}$ | 130,9 ± 5,8 | 133,1 ± 6,9 | 135,9 ± 8,1 | 136,8 ± 7,6 | 137,8 ± 9,8 | 139,7 ± 9,4 | 139,7 ±10,0 | 137,8 ±12,9 |
| $AP_{diast}$ | 81,6 ± 2,8 | 83,4 ± 3,2 | 85,9 ± 3,3 | 88,5 ± 3,7 | 92,8 ± 5,6 | 95,2 ± 4,7 | 92,8 ± 5,6 | 90,9 ± 6,4 |
| PRV | 2,34 ±0,66 | 2,15 ±0,60 | 2,25 ±0,65 | 2,40 ±0,65 | 3,19 ±0,83 | 3,47 ±0,85 | 3,97 ±0,79 | 4,22 ±0,80 |
| $\overline{PAP}$ | 12,81 ±0,78 | 13,34 ±0,71 | 13,37 ±0,58 | 13,87 ±0,52 | 13,81 ±0,28 | 13,5 ±0,46 | 14,41 ±0,39 | 14,41 ±0,62 |
| LVEDP | 5,72 ±0,28 | 6,0 ±0,34 | 6,47 ±0,54 | 8,06 ±0,77 | 9,47 ±0,60 | 9,47 ±0,65 | 10,12 ±0,66 | 10,5 ±0,81 |
| dp/dt | 2306,2 ±346,6 | 2193,7 ±322,0 | 2006,2 ±244,2 | 1968,7 ±297,4 | 1743,7 ±277,9 | 1659,4 ±303,2 | 1640,6 ±264,4 | 1621,9 ±228,8 |
| $HL_L$ | 0,45 ±0,06 | 0,43 ±0,05 | 0,41 ±0,05 | 0,39 ±0,05 | 0,34 ±0,04 | 0,35 ±0,04 | 0,35 ±0,04 | 0,37 ±0,05 |
| $HL_R$ | 50,5 ± 7,3 | 50,8 ± 7,5 | 47,3 ± 6,4 | 46,0 ± 6,6 | 36,7 ± 4,9 | 34,9 ± 4,8 | 36,9 ± 4,1 | 38,3 ± 2,7 |
| SV | 22,1 ± 3,8 | 21,5 ± 3,2 | 20,5 ± 3,2 | 19,4 ± 3,0 | 17,4 ± 2,8 | 17,0 ± 3,1 | 15,9 ± 2,8 | 15,5 ± 2,1 |
| $BF_{Fem}$ | 54,1 ± 8,8 | 58,5 ±10,7 | 62,2 ±11,9 | 62,6 ±13,2 | 57,4 ±12,4 | 49,9 ±11,2 | 49,5 ±11,2 | 50,9 ±10,3 |
| $W_{Fem}$ | 0,26 ±0,05 | 0,25 ±0,05 | 0,25 ±0,06 | 0,26 ±0,06 | 0,29 ±0,07 | 0,36 ±0,12 | 0,37 ±0,13 | 0,32 ±0,08 |
| HZV | 2,18 ±0,29 | 2,05 ±0,25 | 1,93 ±0,25 | 1,82 ±0,25 | 1,57 ±0,20 | 1,57 ±0,19 | 1,61 ±0,20 | 1,73 ±0,17 |
| $W_{AP}$ | 0,45 ±0,07 | 0,50 ±0,06 | 0,50 ±0,08 | 0,46 ±0,09 | 0,39 ±0,08 | 0,35 ±0,04 | 0,37 ±0,05 | 0,32 ±0,06 |
| $W_{TP}$ | 6,10 ±0,68 | 6,62 ±0,75 | 7,24 ±0,87 | 7,90 ±1,09 | 9,39 ±1,45 | 9,56 ±1,45 | 9,10 ±1,37 | 8,13 ±0,95 |

Tabelle VIII : Verbindung gem. Beispiel 9c

| | v | 2 | 5 | 7 | 10 | 15 | 20 | 25 |
|---|---|---|---|---|---|---|---|---|
| HF | 102,5 ± 5,9 | 163,2 ±14,9 | 129,7 ± 2,3 | 123,2 ± 3,3 | 120,5 ± 3,7 | 115,2 ± 3,9 | 114,5 ± 3,1 | 112,5 ± 3,2 |
| $AP_{syst}$ | 147,2 ± 9,8 | 105,0 ±13,3 | 126,6 ± 5,7 | 129,4 ± 5,6 | 127,5 ± 6,8 | 128,4 ± 6,2 | 128,4 ± 7,5 | 131,2 ± 7,9 |
| $AP_{diast}$ | 78,7 ± 6,5 | 58,1 ± 6,9 | 79,7 ± 3,2 | 82,5 ± 4,3 | 81,6 ± 4,9 | 81,6 ± 3,9 | 82,5 ± 5,5 | 83,4 ± 4,9 |
| PRV | 3,0 ±0,53 | 0,90 ±1,01 | 0,88 ±0,69 | 1,16 ±0,55 | 1,12 ±0,55 | 1,63 ±0,53 | 1,41 ±0,47 | 1,46 ±0,46 |
| $\overline{PAP}$ | 13,6 ± 0,8 | 10,6 ± 0,4 | 11,6 ± 0,7 | 11,5 ± 0,4 | 12,0 ± 0,9 | 11,8 ± 0,8 | 11,8 ± 0,9 | 11,4 ± 1,0 |
| LVEDP | 7,60 ±1,0 | 3,60 ±0,87 | 4,64 ±1,35 | 4,92 ±1,15 | 4,92 ±1,20 | 5,20 ±1,10 | 5,48 ±1,18 | 5,62 ±1,12 |
| dp/dt | 1837,5 ±195,4 | 3045,0 ±412,3 | 2287,5 ±191,8 | 2325,0 ±214,9 | 2306,2 ±197,5 | 2250,0 ±188,1 | 2221,9 ±223,5 | 2221,9 ±252,7 |
| $HL_L$ | 0,56 ±0,09 | 0,51 ±0,12 | 0,56 ±0,10 | 0,58 ±0,09 | 0,57 ±0,10 | 0,57 ±0,08 | 0,55 ±0,09 | 0,56 ±0,10 |
| $HL_R$ | 63,08 ±11,5 | 67,6 ±8,2 | 66,9 ±13,8 | 64,2 ±10,2 | 68,8 ±14,8 | 63,6 ±9,8 | 63,1 ±12,1 | 60,5 ±12,8 |
| SV | 25,9 ± 3,2 | 20,0 ± 3,2 | 21,1 ± 2,7 | 22,4 ± 2,5 | 23,0 ± 2,3 | 24,2 ± 2,5 | 23,3 ± 2,4 | 23,7 ± 3,0 |
| $BF_{Fem}$ | 48,0 ± 6,9 | 34,6 ±10,1 | 39,1 ± 9,1 | 42,5 ±10,4 | 41,9 ± 6,1 | 43,3 ± 6,4 | 44,2 ± 6,2 | 41,3 ± 6,3 |
| $W_{Fem}$ | 0,29 ±0,04 | 0,33 ±0,08 | 0,37 ±0,08 | 0,34 ±0,06 | 0,32 ±0,04 | 0,31 ±0,04 | 0,31 ±0,04 | 0,33 ±0,03 |
| HZV | 2,62 ±0,28 | 3,15 ±0,32 | 2,73 ±0,32 | 2,75 ±0,27 | 2,76 ±0,27 | 2,77 ±0,24 | 2,65 ±0,25 | 2,65 ±0,28 |
| $W_{AP}$ | 0,29 ±0,06 | 0,30 ±0,04 | 0,33 ±0,06 | 0,31 ±0,04 | 0,32 ±0,06 | 0,30 ±0,05 | 0,30 ±0,06 | 0,27 ±0,07 |
| $W_{TP}$ | 5,15 ±0,65 | 3,07 ±0,18 | 4,72 ±0,35 | 4,77 ±0,25 | 4,68 ±0,23 | 4,65 ±0,23 | 4,90 ±0,26 | 5,00 ±0,29 |

Tabelle VIII (Fortsetzung) : Verbindung gem. Beispiel 9c

| | 30 | 40 | 50 | 60 | 90 | 120 | 150 | 180 |
|---|---|---|---|---|---|---|---|---|
| HF | 111,5 ± 3,0 | 109,2 ± 2,5 | 105,5 ± 3,2 | 103,2 ± 3,5 | 98,0 ± 3,4 | 96,7 ± 2,0 | 98,2 ± 1,2 | 100,0 ± 1,8 |
| $AP_{syst}$ | 134,1 ± 7,2 | 136,9 ± 8,3 | 142,5 ± 8,5 | 144,7 ± 8,2 | 148,1 ± 7,6 | 150,0 ± 7,6 | 148,7 ± 6,9 | 148,1 ± 5,8 |
| $AP_{diast}$ | 81,6 ± 4,9 | 86,2 ± 5,5 | 88,2 ± 5,6 | 88,2 ± 4,9 | 90,9 ± 3,9 | 90,9 ± 3,5 | 88,7 ± 3,2 | 87,2 ± 3,5 |
| PRV | 1,65 ±0,63 | 1,63 ±0,38 | 1,78 ±0,62 | 1,97 ±0,64 | 2,53 ±0,73 | 3,0 ±0,85 | 3,10 ±0,67 | 3,28 ±0,77 |
| $\overline{PAP}$ | 11,5 ± 0,8 | 11,8 ± 1,0 | 11,8 ± 1,0 | 12,0 ± 0,9 | 13,3 ± 1,2 | 14,2 ± 1,6 | 15,0 ± 1,7 | 15,5 ± 2,0 |
| LVEDP | 5,62 ±1,0 | 5,62 ±0,89 | 6,19 ±0,98 | 6,47 ±1,04 | 7,03 ±1,05 | 7,73 ±1,15 | 7,87 ±1,07 | 7,87 ±1,0 |
| dp/dt | 2221,9 ±243,7 | 2203,1 ±255,0 | 2165,6 ±262,7 | 2109,4 ±229,3 | 1987,5 ±264,3 | 1837,5 ±213,2 | 1809,4 ±236,4 | 1743,7 ±176,9 |
| $HL_L$ | 0,57 ±0,09 | 0,60 ±0,10 | 0,61 ±0,10 | 0,59 ±0,09 | 0,56 ±0,09 | 0,55 ±0,08 | 0,54 ±0,06 | 0,55 ±0,05 |
| $HL_R$ | 60,5 ±11,7 | 63,2 ±12,7 | 61,7 ±12,2 | 59,1 ±11,0 | 59,2 ±10,8 | 60,8 ±10,8 | 64,4 ±10,3 | 67,5 ± 8,6 |
| SV | 24,2 ± 2,5 | 24,9 ± 2,5 | 25,5 ± 2,7 | 25,1 ± 2,5 | 24,8 ± 2,5 | 24,7 ± 2,1 | 24,3 ± 1,8 | 24,8 ± 1,3 |
| $BF_{Fem}$ | 44,2 ± 6,6 | 44,2 ± 5,8 | 46,6 ± 6,2 | 45,8 ± 6,8 | 41,7 ± 6,7 | 39,9 ± 5,6 | 39,0 ± 9,5 | 42,8 ± 4,8 |
| $W_{Fem}$ | 0,31 ±0,03 | 0,32 ±0,03 | 0,31 ±0,03 | 0,32 ±0,03 | 0,36 ±0,04 | 0,38 ±0,04 | 0,37 ±0,03 | 0,33 ±0,03 |
| HZV | 2,68 ±0,23 | 2,71 ±0,26 | 2,68 ±0,25 | 2,59 ±0,24 | 2,42 ±0,24 | 2,39 ±0,20 | 2,38 ±0,15 | 2,47 ±0,09 |
| $W_{AP}$ | 0,28 ±0,06 | 0,30 ±0,06 | 0,26 ±0,07 | 0,27 ±0,07 | 0,33 ±0,08 | 0,35 ±0,10 | 0,40 ±0,11 | 0,41 ±0,13 |
| $W_{TP}$ | 4,87 ±0,17 | 5.05 ±0,21 | 5,24 ±0,18 | 5,48 ±0,29 | 6,00 ±0,31 | 6,07 ±0,26 | 5,92 ±0,15 | 5,61 ±0,04 |

Zur orientierenden Prüfung der akuten Toxizität einiger der erfindungsgemäßen Verbindungen wurden diese in physiologischer Kochsalzlösung an weiblichen NMRI-Albinomäusen intravenös in Dosen von 50, 100 und 200 mg/kg appliziert. Die Substanzen wurden an mindestens 3 Tieren pro Dosis gespritzt. Wenn nach der höchsten applizierten Dosis noch kein Tier gestorben war, wurden keine weiteren Substanzdosen geprüft. Im Zweifelsfall wurde die Prüfung an mindestens 3 weiteren Tieren mit der gleichen Dosis wiederholt. Beobachtet wurde die Todesrate innerhalb 24 Stdn. nach der Applikation.

In der Tabelle IX sind die ermittelten Todesraten und der daraus abgeschätzte $LD_{50}$-Bereich dargestellt.

Tabelle IX

| Beispiel Nr. | Todeshäufigkeit bei der i.v.Dosis v. 200 mg/kg | 100 mg/kg | 50 mg/kg | Geschätzter $LD_{50}$-Bereich (mg/kg) |
|---|---|---|---|---|
| 2 | 1/3 | 0/3 | - | ≥ 200 |
| 3 | 0/6 | - | - | > 200 |
| 4 | 10/10 | 0/10 | - | 100 - 200 |
| 5 | 10/10 | 4/10 | 0/10 | ≥ 100 |
| 7c | 0/6 | - | - | > 200 |
| 8c | 11/13 | 0/3 | - | 100 - 200 |
| 10 c | 0/6 | - | - | > 200 |
| 11 d | 0/6 | - | - | > 200 |
| 12 d | 0/6 | - | - | > 200 |
| 13 | 0/6 | - | - | > 200 |
| 15 | 3/3 | 3/3 | 3/3 | < 50 |
| 16 | 10/10 | 0/10 | - | 100 - 200 |
| 17 | 3/3 | 2/3 | 0/10 | 50 - 100 |

Für Beispiel 1d wurden an einer größeren Zahl von Mäusen folgende Werte für die akute Toxizität nach der Methode von Lichtfield und Wilcoxon ermittelt:

$LD_{50}$ i. v. = 219 mg/kg (190.4-251.9)
$LD_{50}$ i. p. = 760 mg/kg (678.6-851.2)
$LD_{50}$ p. o. = 1 600 mg/kg.

## Ansprüche

1. Durch Purinbasen substituierte Alkylamino-desoxy-1.4 ; 3.6-dianhydro-hexit-nitrate der allgemeinen Formel I,

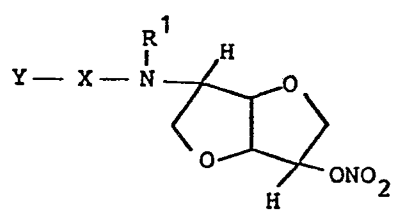

(I)

worin

$R^1$ ein Wasserstoffatom oder eine Niedrigalkylgruppe mit 1 bis 4 C-Atomen,

X eine geradkettige oder verzweigte Alkylen- oder Hydroxyalkylen-Gruppe mit 1 bis 7 C-Atomen,

Y eine 1.3-Dialkylxanthin-7-yl- oder eine 3.7-Dialkyl-xanthin-1-yl-Gruppe, jeweils mit gerad- oder verzweigtkettigen Alkylgruppen mit 1 bis 5 C-Atomen, oder eine Adenin-9-yl-Gruppe

bedeuten, sowie deren physiologisch unbedenkliche Säureadditionssalze.

2. Durch Purinbasen substituierte 5-Alkylamino-5-desoxy-1.4 ; 3.6-dianhydro-L-idit-2-nitrate der allgemeinen Formel V,

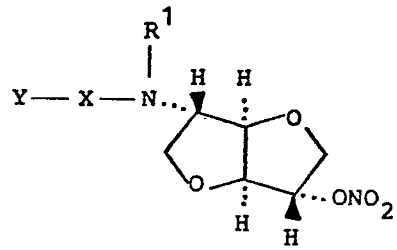

(V)

worin $R^1$, X und Y die in Anspruch 1 angegebenen Bedeutungen besitzen, sowie deren physiologisch unbedenkliche Säureadditionssalze.

3. Durch Purinbasen substituierte 5-Alkylamino-5-desoxy-1.4 ; 3.6-dianhydro-D-glucit-2-nitrate der allgemeinen Formel VI,

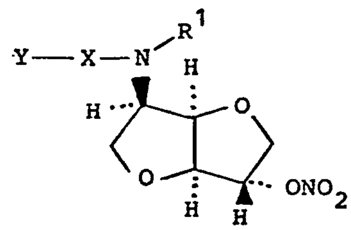

(VI)

worin $R^1$, X und Y die in Anspruch 1 angegebenen Bedeutungen besitzen, sowie deren physiologisch unbedenkliche Säureadditionssalze.

4. Durch Purinbasen substituierte 2-Alkylamino-2-desoxy-1.4 ; 3.6-dianhydro-D-glucit-5-nitrate der allgemeinen Formel VII,

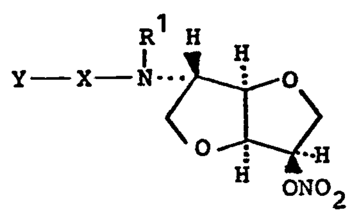

(VII)

worin $R^1$, X und Y die Anspruch 1 angegebenen Bedeutungen besitzen, sowie deren physiologisch unbedenkliche Säureadditionssalze.

5. Durch Purinbasen substituierte 5-Alkylamino-5-desoxy-1.4 ; 3.6-dianhydro-D-mannit-2-nitrate der allgemeinen Formel VIII,

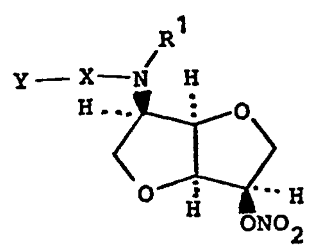

(VIII)

worin $R^1$, X und Y die in Anspruch 1 angegebenen Bedeutungen besitzen, sowie deren physiologisch unbedenkliche Säureadditionssalze.

6. 5-(3-Theophyllin-7-ylpropyl)-amino-5-desoxy-1.4 ; 3.6-dianhydro-L-idit-2-nitrat.

7. 5-(2-Theophyllin-7-yläthyl)-amino-5-desoxy-1.4 ; 3.6-dianhydro-L-idit-2-nitrat.

8. 5-(4-Theophyllin-7-ylbutyl)-amino-5-desoxy-1.4 ; 3.6-dianhydro-L-idit-2-nitrat.

9. 5-(5-Theophyllin-7-ylpentyl)-amino-5-desoxy-1.4 ; 3.6-dianhydro-L-idit-2-nitrat.

10. 5-(6-Theophyllin-7-ylhexyl)-amino-5-desoxy-1.4 ; 3.6-dianhydro-L-idit-2-nitrat.

11. 5-(2-Hydroxy-3-theophyllin-7-ylpropyl)-amino-5-desoxy-1.4 ; 3.6-dianhydro-L-idit-2-nitrat.

12. 5-[3-(3.7-Dimethylxanthin-1-yl)-propylamino]-5-desoxy-1.4 ; 3.6-dianhydro-L-idit-2-nitrat.

13. 5-[3-(3-Isobutyl-1-methylxanthin-7-yl)-propylamino]-5-desoxy-1.4 ; 3.6-dianhydro-L-idit-2-nitrat.

14. 5-[N-methyl-N-(3-theophyllin-7-ylpropyl)-amino]-5-desoxy-1.4 ; 3.6-dianhydro-L-idit-2-nitrat.

15. 5-(2-Adenin-9-yläthyl)-amino-5-desoxy-1.4 ; 3.6-dianhydro-L-idit-2-nitrat.

16. 5-(3-Adenin-9-ylpropyl)-amino-5-desoxy-1.4 ; 3.6-dianhydro-L-idit-2-nitrat.
17. 5-(4-Adenin-9-ylbutyl)-amino-5-desoxy-1.4 ; 3.6-dianhydro-L-idit-2-nitrat.
18. 5-(5-Adenin-9-ylpentyl)-amino-5-desoxy-1.4 ; 3.6-dianhydro-L-idit-2-nitrat.
19. 5-(6-Adenin-9-ylhexyl)-amino-5-desoxy-1.4 ; 3.6-dianhydro-L-idit-2-nitrat.
20. 5-[1-Methyl-3-(theophyllin-7-yl)-propylamino]-5-desoxy-1.4 ; 3.6-dianhydro-L-idit-2-nitrat.
21. (+)-5-[1-Methyl-3-(theophyllin-7-yl)-propylamino]-5-desoxy-1.4 ; 3.6-dianhydro-L-idit-2-nitrat.
22. (−)-5-[1-Methyl-3-(theophyllin-7-yl)-propylamino]-5-desoxy-1.4 ; 3.6-dianhydro-L-idit-2-nitrat.
23. 5-[1-Methyl-5-(theophyllin-7-yl)-pentylamino]-desoxy-1.4 ; 3.6-dianhydro-L-idit-2-nitrat.
24. 5-(3-Theophyllin-7-ylpropyl)-amino-5-desoxy-1.4 ; 3.6-dianhydro-D-glucit-2-nitrat.
25. 2-(3-Theophyllin-7-ylpropyl)-amino-2-desoxy-1.4 ; 3.6-dianhydro-D-glucit-5-nitrat.
26. 5-(3-Theophyllin-7-ylpropyl)-amino-5-desoxy-1.4 ; 3.6-dianhydro-D-mannit-2-nitrat.
27. Verfahren zur Herstellung der Verbindungen gemäß Anspruch 1, dadurch gekennzeichnet, daß

(A) L-Isoidid, D-Isosorbid oder D-Isomannid mit einem Sulfonsäurechlorid in den entsprechenden Mono-O-acyl-1.4 ; 3.6-dianhydro-hexit übergeführt wird,

(B) welcher durch Aminolyse mit wäßriger Ammoniaklösung oder einem primären Alkylamin mit 1 bis 4 C-Atomen, gegebenenfalls unter Zusatz eines geeigneten Lösungsmittels, zum entsprechenden Amino- bzw. Alkylamino-desoxy-1.4 ; 3.6-dianhydrohexit umgesetzt wird,

(C) wonach die freie Hydroxylgruppe des entstandenen Amino-desoxy-1.4 ; 3.6-dianhydro-hexits oder gegebenenfalls dessen Säureadditionssalzes mit Salpetersäure, Nitriersäure oder mit einem Gemisch aus Salpetersäure und Eisessig/Acetanhydrid in Gegenwart von Harnstoff zum entsprechenden Amino-desoxy-1.4 ; 3.6-dianhydro-hexit-nitrat verestert wird, und

(D) anschließend das Nitrat mit dem entsprechenden ω-Halogenalkyl-, Epoxyalkyl- oder ω-Acyloxyalkylxanthin- oder -adenin-Derivat, gegebenenfalls in Gegenwart einer Hilfsbase, zum gewünschten Endprodukt umgesetzt wird.

28. Verfahren zur Herstellung der Verbindungen gemäß Anspruch 1, dadurch gekennzeichnet, daß der in Stufe (B) des Verfahrens gemäß Anspruch 27 entstehende Amino- bzw. Alkylamino-desoxy-1.4 ; 3.6-dianhydro-hexit zuerst mit dem entsprechenden ω-Halogenalkyl-, Epoxyalkyl- oder ω-Acyloxyalkylxanthin- oder -adenin-Derivat, gegebenenfalls in Gegenwart einer Hilfsbase, umgesetzt wird und erst danach die freie Hydroxylgruppe des entstandenen Purinylalkyl-amino-desoxy-1.4 ; 3.6-dianhydro-hexit-Derivats, oder gegebenenfalls dessen Säureadditionssalzes, mit Salpetersäure, Nitriersäure oder mit einem Gemisch aus Salpetersäure und Eisessig/Acetanhydrid, in Gegenwart von Harnstoff zum entsprechenden Purinyl-alkyl-amino-desoxy-1.4 ; 3.6-dianhydro-hexit-nitrat verestert wird.

29. Verfahren nach Anspruch 28, dadurch gekennzeichnet, daß der Aminstickstoff des Aminoisohexids vor der Umsetzung mit dem reaktiven Purinderivat mit einer Schutzgruppe, die durch katalytische Hydrierung leicht abgespalten werden kann, gegen Disubstitution geschützt wird.

30. Verfahren zur Herstellung der Verbindungen gemäß Anspruch 1, dadurch gekennzeichnet, daß der gemäß Stufe (B) des Verfahrens gemäß Anspruch 28 hergestellte Amino- bzw. Alkylamino-desoxy-1.4 ; 3.6-dianhydro-hexit mit einem entsprechenden ω-Acylalkyl- bzw. ω-Oxoalkyl-xanthin- oder -adenin-Derivat versetzt und in an sich bekannter Weise einer reduktiven Kondensation in Gegenwart von Wasserstoff und einem geeigneten Katalysator, gegebenenfalls in Gegenwart eines Lösungsmittels, unterworfen wird.

31. Verfahren zur Herstellung der Verbindungen gemäß Anspruch 2, dadurch gekennzeichnet, daß

(A') D-Isosorbid mit einem Überschuß an Sulfonsäurechlorid quantitativ zum entsprechenden Isosorbiddiacylat umgesetzt wird,

(B') welches durch selektive Aminolyse mit wäßriger Ammoniaklösung oder einem primären Alkylamin mit 1 bis 4 C-Atomen, gegebenenfalls unter Zusatz eines geeigneten Lösungsmittels, zum 5-Amino- bzw. 5 Alkylamino-5-desoxy-1.4 ; 3.6-dianhydro-L-idit-2-sulfonat umgesetzt wird,

(C') wonach das nach (B') erhaltene Reaktionsgemisch einer sauren oder alkalischen Hydrolyse unterworfen wird und

(D') der so entstandene 5-Amino- bzw. 5-Alkylamino-5-desoxy-1.4 ; 3.6-dianhydro-L-idit gemäß Anspruch 27, (C) und (D), bzw. gemäß den Ansprüchen 28 bis 30 weiter aufgearbeitet wird.

32. Verfahren zur Herstellung der Verbindungen gemäß Anspruch 3, dadurch gekennzeichnet, daß

(A'') D-Isosorbid mit einem Überschuß an Sulfonsäurechlorid quantitativ zum entsprechenden Isosorbiddiacylat umgesetzt wird,

(B'') welches mit Natriumbenzoat selektiv zum entsprechenden 1.4 ; 3.6-Dianhydro-L-idit-2-sulfonat-5-benzoat umgesetzt wird,

(C'') wonach letzteres durch Ammonolyse mit wäßriger Ammoniaklösung oder durch Aminolyse mit einem primären oder sekundären Alkylamin mit 1 bis 4 C-Atomen, wobei gleichzeitig der Benzoesäurerest hydrolytisch abgespalten wird, zum entsprechenden 5-Amino- bzw. 5-Alkylamino-5-desoxy-1.4 ; 3.6-dianhydro-D-glucit umgesetzt wird und

(D'') das so entstandene Produkt gemäß Anspruch 27 (C) und (D) bzw. gemäß den Ansprüchen 28 bis 30 weiter aufgearbeitet wird.

33. Verfahren nach Anspruch 31 oder 32, dadurch gekennzeichnet, daß als Sulfonsäurechlorid Methansulfonsäurechlorid oder Toluolsulfonsäurechlorid verwendet wird.

34. Verfahren nach Anspruch 32, dadurch gekennzeichnet, daß die Umsetzung mit Natriumbenzoat

(B") in einem wasserfreien, dipolaren, aprotischen Lösungsmittel unter Inertgasatmosphäre bei erhöhter Temperatur durchgeführt wird.

35. Verfahren nach Anspruch 34, dadurch gekennzeichnet, daß Dimethylformamid als Lösungsmittel verwendet wird und die Umsetzung bei 100 bis 180 °C durchgeführt wird.

36. Verfahren nach Anspruch 27 oder 31, dadurch gekennzeichnet, daß die Aminolyse unter erhöhtem Druck und erhöhter Temperatur durchgeführt wird.

37. Verfahren nach Anspruch 27, dadurch gekennzeichnet, daß die Aminolyse bei einer Temperatur von 90 bis 180 °C durchgeführt wird.

38. Verfahren nach Anspruch 31, dadurch gekennzeichnet, daß die Aminolyse bei einer Temperatur von 90 bis 110 °C durchgeführt wird.

39. Pharmazeutische Zubereitung, enthaltend eine Verbindung gemäß einem der Ansprüche 1 bis 26 sowie übliche Träger und Zusatzstoffe.

## Claims

1. Alkylaminodesoxy-1.4 ; 3.6-dianhydrohexitol nitrates substituted by purine bases of the general formula I,

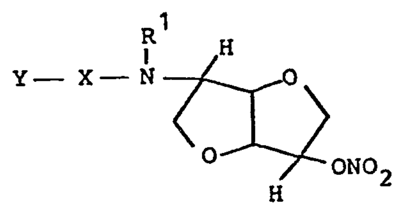

(I)

wherein

$R^1$ signifies a hydrogen atom or a lower alkyl group with 1 to 4 C-atoms,

X a straight-chained or branched alkylene or hydroxyalkylene group with 1 to 7 C-atoms

Y a 1,3-dialkylxanthin-7-yl or a 3,7-dialkylxanthin-1-yl group, each with straight or branched-chained alkyl groups with 1 to 5 C-atoms, or an adenin-9-yl group,

as well as their physiologically acceptable acid-addition salts.

2. 5-Alkylamino-5-desoxy-1.4 ; 3.6-dianhydro-L-iditol 2-nitrates substituted by purine bases of the general formula V,

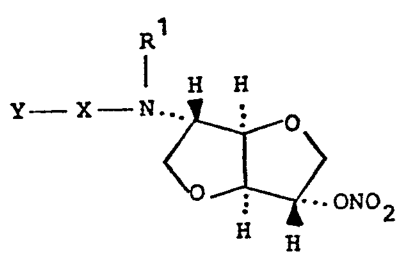

(V)

wherein $R^1$, X and Y possess the meanings given in claim 1, as well as their physiologically acceptable acid-addition salts.

3. 5-Alkylamino-5-desoxy-1.4 ; 3.6-dianhydro-D-glucitol 2-nitrates substituted by purine bases of the general formula VI,

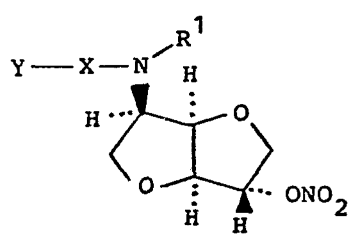

(VI)

wherein $R^1$, X and Y possess the meanings given in claim 1, as well as their physiologically acceptable acid-addition salts.

4. 2-Alkylamino-2-desoxy-1.4 ; 3.6-dianhydro-D-glucitol 5-nitrates substituted by purine bases of the general formula VII,

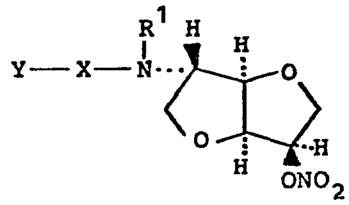

(VII)

wherein $R^1$, X and Y possess the meanings given in claim 1, as well as their physiologically acceptable acid-addition salts.

5. 5-Alkylamino-5-desoxy-1.4 ; 3.6-dianhydro-D-mannitol 2-nitrates substituted by purine bases of the general formula VIII,

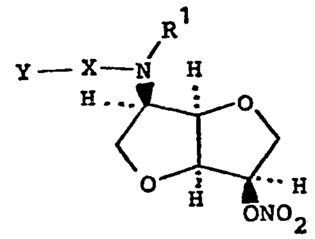

(VIII)

wherein $R^1$, X and Y possess the meanings given in claim 1, as well as their physiologically acceptable acid-addition salts.

6. 5-(3-Theophyllin-7-ylpropyl)-amino-5-desoxy-1.4 ; 3.6-dianhydro-L-iditol 2-nitrate.
7. 5-(2-Theophyllin-7-ylethyl)-amino-5-desoxy-1.4 ; 3.6-dianhydro-L-iditol 2-nitrate.
8. 5-(4-Theophyllin-7-ylbutyl)-amino-5-desoxy-1.4 ; 3.6-dianhydro-L-iditol 2-nitrate.
9. 5-(5-Theophyllin-7-ylpentyl)-amino-5-desoxy-1.4 ; 3.6-dianhydro-L-iditol 2-nitrate.
10. 5-(6-Theophyllin-7-ylhexyl)-amino-5-desoxy-1.4 ; 3.6-dianhydro-L-iditol 2-nitrate.
11. 5-(2-Hydroxy-3-theophyllin-7-ylpropyl)-amino-5-desoxy-1.4 ; 3.6-dianhydro-L-iditol 2-nitrate.
12. 5-[3-(3,7-Dimethylxanthin-1-yl)-propylamino]-5-desoxy-1.4 ; 3.6-dianhydro-L-iditol 2-nitrate.
13. 5-[3-(3-Isobutyl-1-methylxanthin-7-yl)-propylamino]-5-desoxy-1.4 ; 3.6-dianhydro-L-iditol 2-nitrate.
14. 5-[N-Methyl-N-(3-theophyllin-7-ylpropyl)-amino]-5-desoxy-1.4 ; 3.6-dianhydro-L-iditol 2-nitrate.
15. 5-(2-Adenin-9-ylethyl)-amino-5-desoxy-1.4 ; 3.6-dianhydro-L-iditol 2-nitrate.
16. 5-(3-Adenin-9-ylpropyl)-amino-5-desoxy-1.4 ; 3.6-dianhydro-L-iditol 2-nitrate.
17. 5-(4-Adenin-9-ylbutyl)-amino-5-desoxy-1.4 ; 3.6-dianhydro-L-iditol 2-nitrate.
18. 5-(5-Adenin-9-ylpentyl)-amino-5-desoxy-1.4 ; 3.6-dianhydro-L-iditol 2-nitrate.
19. 5-(6-Adenin-9-ylhexyl)-amino-5-desoxyl-1.4 ; 3.6-dianhydro-L-iditol 2-nitrate.
20. 5-[1-Methyl-3-(theophyllin-7-yl)-propylamino]-5-desoxy-1.4 ; 3.6-dianhydro-L-iditol 2-nitrate.
21. (+)-5-[1-Methyl-3-(theophyllin-7-yl)-propylamino]-5-desoxy-1.4 ; 3.6-dianhydro-L-iditol 2-nitrate.
22. (−)-5-[1-Methyl-3-(theophyllin-7-yl)-propylamino]-5-desoxy-1.4 ; 3.6-dianhydro-L-iditol 2-nitrate.
23. 5-[1-Methyl-5-(theophyllin-7-yl)-pentylamino]-desoxy-1.4 ; 3.6-dianhydro-L-iditol 2-nitrate.
24. 5-(3-Theophyllin-7-ylpropyl)-amino-5-desoxy-1.4 ; 3.6-dianhydro-D-glucitol 2-nitrate.
25. 2-(3-Theophyllin-7-ylpropyl)-amino-2-desoxy-1.4 ; 3.6-dianhydro-D-glucitol 5-nitrate.
26. 5-(3-Theophyllin-7-ylpropyl)-amino-5-desoxy-1.4 ; 3.6-dianhydro-D-mannitol 2-nitrate.
27. Process for the preparation of compounds according to claim 1, characterised in that

(A) L-isoidide, D-isosorbide or D-isomannide is converted with a sulphonic acid chloride into the corresponding mono-O-acyl-1.4 ; 3.6-dianhydrohexitol,

(B) which, by aminolysis with aqueous ammonia solution or a primary alkylamine with 1 to 4 C-atoms, is reacted to give the corresponding amino- or alkylamino-desoxy-1.4 ; 3.6-dianhydrohexitol, possibly with the addition of a suitable solvent,

(C) whereafter the free hydroxyl group of the resultant aminodesoxy-1.4 ; 3.6-dianhydrohexitol or possibly of its acid-addition salt is esterified with nitric acid, nitrating acid or with a mixture of nitric acid and glacial acetic acid/acetic anhydride in the presence of urea to give the corresponding aminodesoxy-1.4 ; 3.6-dianhydrohexitol nitrate, and

(D) subsequently the nitrate is reacted with the corresponding ω-haloalkyl, epoxyalkyl or ω-acyloxyalkylxanthine or adenine derivative, possibly in the presence of an adjuvant base, to give the desired end product.

28. Process for the preparation of compounds according to claim 1, characterised in that the amino- or alkylamino-desoxy-1.4 ; 3.6-dianhydrohexitol resulting in step (B) of the process according to claim 27 is first reacted with the corresponding ω-haloalkyl-, epoxyalkyl- or ω-acyloxyalkyl-xanthine or adenine derivative, possibly in the presence of an adjuvant base, and only thereafter is the free hydroxyl group of the resultant purinylalkylaminodesoxy-1.4 ; 3.6-dianhydrohexitol derivative or possibly its acid-addition salt esterified with nitric acid, nitrating acid or with a mixture of nitric acid and glacial acetic acid/acetic

anhydride, in the presence of urea to give the corresponding purinylalkylaminodesoxy-1.4 ; 3.6-dianhydrohexitol nitrate.

29. Process according to claim 28, characterised in that the amine nitrogen of the aminisohexide is, before the reaction with the reactive purine derivative, protected against disubstitution with a protective group which can easily be split off by catalytic hydrogenation.

30. Process for the preparation of compounds according to claim 1, characterised in that the amino- or alkylaminodesoxy-1.4 ; 3.6-dianhydrohexitol prepared according to step (B) of the process according to claim 28 is mixed with an appropriate ω-acylalkyl- or ω-oxoalkylxanthine or -adenine derivative and subjected in *per se* known manner to a reductive condensation in the presence of hydrogen and a suitable catalyst, possibly in the presence of a solvent.

31. Process for the preparation of compounds according to claim 2, characterised in that

(A') D-isosorbide is reacted quantitatively with an excess of sulphonic acid chloride, to give the corresponding isosorbide diacylate,

(B') which, by selective aminolysis with aqueous ammonia solution or a primary alkylamine with 1 to 4 C-atoms, possibly with the addition of a suitable solvent, is reacted to give the 5-amino- or 5-alkylamino-5- desoxy-1.4 ; 3.6-dianhydro-L-iditol 2-sulphonate,

(C') whereafter the reaction mixture obtained under (B') is subjected to an acidic or alkaline hydrolysis and

(D') the thus resulting 5-amino- or 5-alkylamino-5-desoxy-1.4 ; 3.6-dianhydro-L-iditol is further worked up according to claim 27, (C) and (D) or according to claims 28-30.

32. Process for the preparation of compounds according to claim 3, characterised in that

(A") D-isosorbide is reacted quantitatively with an excess of sulphonic acid chloride to give the corresponding isosorbide diacylate,

(B") which is selectively reacted with sodium benzoate to give the corresponding 1.4 ; 3.6-dianhydro-L-iditol 2-sulphonate 5-benzoate,

(C") whereafter the latter is reacted by ammonolysis with aqueous ammonia solution or by aminolysis with a primary or secondary alkylamine with 1 to 4 C-atoms, whereby the benzoic acid residue is simultaneously split off hydrolytically, to give the corresponding 5-amino- or 5-alkylamino-5-desoxy-1.4 ; 3.6-dianhydro-D-glucitol and

(D") the so resulting product is further worked up according to claim 27 (C) and (D) or according to claims 28-30.

33. Process according to claim 31 or 32, characterised in that methanesulphonyl chloride or toluenesulphonyl chloride is used as said sulphonic acid chloride.

34. Process according to claim 32, characterised in that the reaction with sodium benzoate (B") is carried out in an anhydrous, dipolar, aprotic solvent under an inert gas atmosphere at an elevated temperature.

35. Process according to claim 34, characterised in that dimethylformamide is used as said solvent and that the reaction is carried out at 100 to 180 °C.

36. Process according to claim 27 or 31, characterised in that the aminolysis is carried out under elevated pressure and elevated temperature.

37. Process according to claim 27, characterised in that said aminolysis is carried out at a temperature of from 90 to 180 °C.

38. Process according to claim 31, characterised in that the aminolysis is carried out at a temperature of from 90 to 110 °C.

39. Pharmaceutical composition, containing a compound according to one of claims 1 to 26, as well as conventional carrier and additive materials.

**Revendications**

1. Alcoylamino-désoxy-1.4 ; 3.6-dianhydro-hexite-nitrates substitués par des bases puriques de formule générale I,

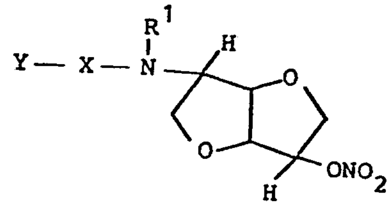

(I)

où

$R^1$ représente un atome d'hydrogène ou un groupe alcoyle inférieur en $C_1$ à $C_4$,

X représente un groupe alcoylène ou hydroxyalcoylène à chaîne droite ou ramifiée en $C_1$ à $C_7$.

Y représente un groupe 1.3-dialcoylxanthine-7-yle ou un groupe 3.7-dialcoyl-xanthin-1-yle, à chaque

fois avec des groupes alcoyle à chaîne droite ou ramifiée en $C_1$ à $C_5$, ou un groupe adénin-9-yle, ainsi que leurs sels d'addition d'acides physiologiquement acceptables.

2. 5-alcoylamino-5-désoxy-1.4 ; 3.6-dianhydro-L-idite-2-nitrates substitués par des bases puriques de formule générale V,

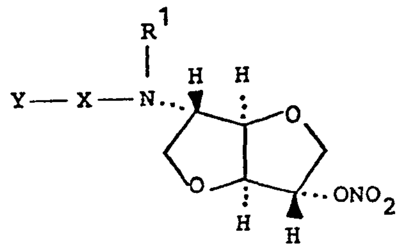

(V)

où $R^1$, X et Y ont les significations données dans la revendication 1 ainsi que leurs sels d'addition d'acides physiologiquement acceptables.

3. 5-alcoylamino-5-désoxy-1.4 ; 3.6-dianhydro-D-glucite-2-nitrates substitués par des bases puriques de formule générale VI,

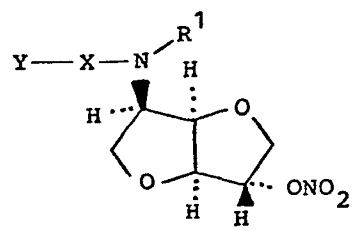

(VI)

où $R^1$, X et Y ont les significations données dans la revendication 1, ainsi que leurs sels d'addition d'acides physiologiquement acceptables.

4. 2-alcoylamino-2-désoxy-1.4 ; 3.6-dianhydro-D-glucite-5-nitrates substitués par des bases puriques de formule générale VII,

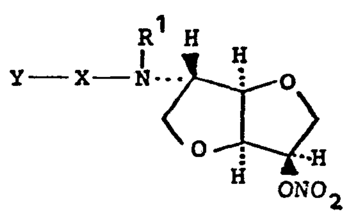

(VII)

où $R^1$, X et Y ont les significations données dans la revendication 1, ainsi que leurs sels d'addition d'acides physiologiquement acceptables.

5. 5-alcoylamino-5-désoxy-1.4 ; 3.6-dianhydro-D-mannite-2-nitrates substitués par des bases puriques de formule générale VIII

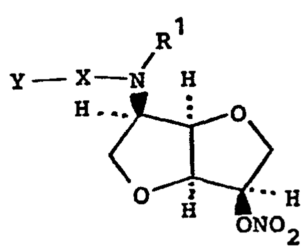

(VIII)

où $R^1$, X et Y ont les significations données dans la revendication 1, ainsi que leurs sels d'addition d'acides physiologiquement acceptables.

6. 5-(3-Théophyllin-7-ylpropyl)-amino-5-désoxy-1.4 ; 3.6-dianhydro-L-idit-2-nitrate.

7. 5-(2-Théophyllin-7-yléthyl)-amino-5-désoxy-1.4 ; 3.6-dianhydro-L-idit-2-nitrate

8. 5-(4-Théophyllin-7-ylbutyl)-amino-5-désoxy-1.4 ; 3.6-dianhydro-L-idit-2-nitrate.

9. 5-(5-Théophyllin-7-ylpentyl)-amino-5-désoxy-1.4 ; 3.6-dianhydro-L-idit-2-nitrate.

10. 5-(6-Théophyllin-7-ylhexyl)-amino-5-désoxy-1.4 ; 3.6-dianhydro-L-idit-2-nitrate.

11. 5-(2-Hydroxy-3-théophyllin-7-ylpropyl)-amino-5-désoxy-1.4 ; 3.6-dianhydro-L-idit-2-nitrate.

12. 5-[3-(3.7-Diméthylxanthin-1-yl)-propylamino]-5-désoxy-1.4 ; 3.6-dianhydro-L-idit-2-nitrate.

13. 5-[3-(Isobutyl-1-méthylxanthin-7-yl)-propylamino]-5-désoxy-1.4 ; 3.6-dianhydro-L-idit-2-nitrate.
14. 5-[N-méthyl-N-(3-théophyllin-7-ylpropyl)-amino]-5-désoxy-1.4 ; 3.6-dianhydro-L-idit-2-nitrate.
15. 5-(2-Adénin-9-yléthyl)-amino-5-désoxy-1.4 ; 3.6-dianhydro-L-idit-2-nitrate.
16. 5-(3-Adénin-9-ylpropyl)-amino-5-désoxy-1.4 ; 3.6-dianhydro-L-idit-2-nitrate.
17. 5-(4-Adénin-9-ylbutyl)-amino-5-désoxy-1.4 ; 3.6-dianhydro-L-idit-2-nitrate.
18. 5-(5-Adénin-9-ylpentyl)-amino-5-désoxy-1.4 ; 3.6-dianhydro-L-idit-2-nitrate.
19. 5-(6-Adénin-9-ylhexyl)-amino-5-désoxy-1.4 ; 3.6-dianhydro-L-idit-2-nitrate.
20. 5-[1-Méthyl-3-(théophyllin-7-yl)-propylamino]-5-désoxy-1.4 ; 3.6-dianhydro-L-idit-2-nitrate.
21. (+)-5-[1-Méthyl-3-(théophyllin-7-yl)-propylamino]-5-désoxy-1.4 ; 3.6-dianhydro-L-idit-2-nitrate.
22. (−)-5-[1-Méthyl-3-(théophyllin-7-yl)propylamino]-5-désoxy-1.4 ; 3.6-dianhydro-L-idit-2-nitrate.
23. 5-[1-Méthyl-5-(théophyllin-7-yl)-pentylamino]-désoxy-1.4 ; 3.6-dianhydro-L-idit-2-nitrate.
24. 5-(3-Théophyllin-7-ylpropyl)-amino-5-désoxy-1.4 ; 3.6-dianhydro-D-glucit-2-nitrate.
25. 2-(3-Théophyllin-7-ylpropyl)-amino-2-désoxy-1.4 ; 3.6-dianhydro-D-glucit-5-nitrat.
26. 5-(3-Théophyllin-7-ylpropyl)-amino-5-désoxy-1.4 ; 3.6-dianhydro-D-mannit-2-nitrate.

27. Procédé de préparation des composés selon la revendication 1, caractérisé en ce que

(A) on transforme le L-isoidide, D-isosorbide ou D-isomannide avec un chlorure d'acide sulfonique en le mono-O-acyl-1.4 ; 3.6-dianhydro-hexite correspondant,

(B) que l'on fait réagir par aminolyse avec une solution aqueuse d'ammoniac ou une alcoylamine primaire en $C_1$ à $C_4$, éventuellement avec addition d'un solvant approprié, pour donner l'amino- ou selon les cas alcoylamino-désoxy-1.4 ; 3.6-dianhydrohexite correspondant,

(C) après quoi on estérifie le groupe hydroxyle libre du amino-désoxy-1.4 ; 3.6-dianhydro-hexite apparu ou éventuellement son sel d'addition d'acide avec de l'acide nitrique, un mélange sulfonitrique ou un mélange d'acide nitrique et d'acide acétique glacial/acétanhydride en présence d'urée pour donner l'aminodésoxy-1.4 ; 3.6-dianhydro-hexite-nitrate correspondant, et

(D) ensuite on fait réagir le nitrate avec le dérivé d'ω-halogènealcoyl-, époxyalcoyl-, ou ω-acyloxyalcoyl-xanthine ou -adénine, éventuellement en présence d'une base auxiliaire, pour donner le produit final désiré.

28. Procédé de préparation des composés selon la revendication 1, caractérisé en ce que dans l'étape (B) du procédé selon la revendication 27 on fait réagir l'amino- ou selon les cas alcoylamino-désoxy-1.4 ; 3.6-dianhydro-hexite apparu tout d'abord avec le dérivé d'ω-halogènealcoyl-, époxyalcoyl- ou -ω-acyl-oxyalcoyl-xanthine ou -adénine, éventuellement en présence d'une base auxiliaire, et seulement alors on estérifie le groupe hydroxyle libre du dérivé de purinyl-alcoyl-amino-désoxy-1.4 ; 3.6-dianhydro-hexite apparu, ou éventuellement son sel d'addition d'acide, avec de l'acide nitrique, un mélange sulfonitrique ou avec un mélange d'acide nitrique et d'acide acétique glacial/acétanhydride, en présence d'urée pour donner le purinyl-alcoyl-amino-désoxy-1.4 ; 3.6-dianhydro-hexite-nitrate.

29. Procédé selon la revendication 28, caractérisé en ce qu'on protège contre une disubstitution l'azote de l'amine de l'aminoisohexide avant la réaction avec le dérivé purique réactif au moyen d'un groupe protecteur que l'on peut facilement séparer par hydrogénation catalytique.

30. Procédé de préparation des composés selon la revendication 1, caractérisé en ce qu'on mélange l'amino- ou selon les cas alcoylamino-désoxy-1.4 ; 3.6-dianhydrohexite préparé selon l'étape (B) du procédé selon la revendication 28 avec un dérivé d'ω-acylalcoyl- ou selon les cas ω-oxoalcoyl-xanthine ou -adénine correspondant et en ce qu'on le soumet de façon connue à une condensation réductrice en présence d'hydrogène et d'un catalyseur approprié, éventuellement en présence d'un solvant.

31. Procédé de préparation des composés selon la revendication 2, caractérisé en ce que

(A') on fait réagir du D-isosorbide avec un excès de chlorure d'acide sulfonique de façon quantitative pour donner le diacylate d'isosorbide correspondant,

(B') que l'on fait réagir par aminolyse sélective avec une solution aqueuse d'ammoniac ou avec une alcoylamine primaire en $C_1$ à $C_4$, éventuellement en ajoutant un solvant approprié, pour donner le 5-amino- ou selon les cas 5-alcoylamino-5-désoxy-1.4 ; 3.6-dianhydro-L-idite-2-sulfonate,

(C') après quoi on soumet le mélange réactionnel obtenu selon (B') à une hydrolyse acide ou alcaline et

(D') on traite plus avant le 5-amino- ou selon les cas 5-alcoylamino-5-désoxy-1.4 ; 3.6-dianhydro-L-idite ainsi apparu selon la revendication 27,

(C) et (D) ou selon les cas, selon les revendications 28 à 30.

32. Procédé de préparation des composés selon la revendication 3, caractérisé en ce que

(A'') on fait réagir du D-isosorbide avec un excès de chlorure d'acide sulfonique de façon quantitative pour donner le diacylate d'isosorbide correspondant,

(B'') que l'on fait réagir de façon sélective avec du benzoate de sodium pour donner le 1.4 ; 3.6-dianhydro-L-idite-2-sulfonate-5-benzoate correspondant,

(C'') après quoi on fait réagir ce dernier par ammonolyse avec une solution aqueuse d'ammoniac ou par aminolyse avec une alcoylamine primaire ou secondaire en $C_1$ à $C_4$, où l'on sépare simultanément le radical acide benzoïque par hydrolyse, pour donner le 5-amino- ou selon les cas 5-alcoylamino-5-désoxy-1.4 ; 3.6-dianhydro-D-glucite correspondant, et

(D'') on traite plus avant le produit ainsi apparu selon la revendication 27 (C) et (D) ou selon les cas selon les revendications 28 à 30.

33. Procédé selon les revendications 31 et 32, caractérisé en ce qu'on utilise comme chlorure d'acide sulfonique le chlorure de l'acide méthanesulfonique ou le chlorure de l'acide toluènesulfonique.

34. Procédé selon la revendication 32, caractérisé en ce qu'on conduit la réaction avec du benzoate de sodium (B") dans un solvant aprotique dipolaire anhydre dans une atmosphère de gaz inerte à température augmentée.

35. Procédé selon la revendication 34, caractérisé en ce qu'on utilise du diméthylformamide comme solvant et en ce qu'on conduit la réaction à 100 à 180 °C.

36. Procédé selon les revendications 27 ou 31, caractérisé en ce qu'on conduit à l'aminolyse à pression accrue et à température augmentée.

37. Procédé selon la revendication 27, caractérisé en ce qu'on conduit l'aminolyse à une température de 90 à 180 °C.

38. Procédé selon la revendication 31, caractérisé en ce qu'on conduit l'aminolyse à une température de 90 à 110 °C.

39. Préparation pharmaceutique contenant un composé selon l'une des revendications 1 à 26 ainsi que les supports et additifs habituels.